# EUROPEAN PATENT SPECIFICATION

(11) **EP 1 935 976 B1**
(45) Date of publication and mention of the grant of the patent: **15.02.2012**
(21) Application number: 07021593.4
(22) Date of filing: 24.02.2006
(51) Int. Cl.: C12N 5/07, A61K 48/00, A61K 35/14

(54) **Transfected myeloid-like cells for the treatment of retinopathy of prematurity and related diseases**
Transfizierte myeloid-ähnliche Zellen zur Behandlung von Frühgeborenenretinopathie und damit zusammenhängenden Netzhauterkrankungen
Cellules myeloides transfectées pour le traitement de rétinopathie de prématurité et maladies rétinopathiques associées

(30) Priority: 24.02.2005 US 656122 P
(43) Date of publication of application: 25.06.2008
(62) Divisional of application: 06736134.5
(73) Proprietor: THE SCRIPPS RESEARCH INSTITUTE, La Jolla, CA 92037 (US)
(72) Inventor: Friedlander, Martin, Del Mar California 92014 (US); Banin, Eyal, La Jolla California 92037 (US); Aguilar, Edith, San Diego California 92126 (US)
(74) Representative: Schnappauf, Georg

(56) References cited:
- WO-A-2004/010959
- WO-A-2006/031467
- US-A1- 2007 231 310

## Description

### CROSS-REFERENCE TO RELATED APPLICATIONS

This application claims the benefit of U.S. Provisional Patent Application Serial No. 60/656,122, filed on February 24, 2005.

### STATEMENT OF GOVERNMENT INTEREST

A portion of the work described herein was supported by grants number EY11254 and EY12598 from the National Eye Institute of the National Institutes of Health. The United States Government has certain rights in this invention.

### FIELD OF THE INVENTION

The technical field of invention relates to the treatment of retinopathic diseases. More particularly, it relates to the treatment of retinopathic diseases such as retinopathy of prematurity by administering cells as specified herein to the eye of a mammal suffering from or at risk of developing said diseases.

### BACKGROUND OF THE INVENTION

Vascular diseases of the retina, including diabetic retinopathy, exudative age related macular degeneration (ARMD), retinopathy of prematurity (ROP) and vascular occlusions, are major causes of visual impairment and blindness. This group of diseases is the focus of intense research aimed to identify novel treatment modalities that will help prevent or modify pathological ocular neovascularization. For example, ARMD affects 12-15 million American over the age of 65 and causes visual loss in 10-15% of them as a direct effect of choroidal (sub-retinal) neovascularization. The leading cause of visual loss for Americans under the age of 65 is diabetes; 16 million individuals in the United States are diabetic and 40,000 per year suffer from ocular complications of the disease, often a result of retinal neovascularization. While laser photocoagulation has been effective in preventing severe visual loss in subgroups of high risk diabetic patients, the overall 10-year incidence of retinopathy remains substantially unchanged. For patients with choroidal neovascularization due to ARMD or inflammatory eye disease such as ocular histoplasmosis, photocoagulation, with few exceptions, is ineffective in preventing visual loss. While recently developed, non-destructive photodynamic therapies hold promise for temporarily reducing individual loss in patients with previously untreatable choroidal neovascularization, only 61.4% of patients treated every 3-4 months had improved or stabilized vision compared to 45.9% of the placebo-treated group.

Age related macular degeneration and diabetic retinopathy are the leading causes of visual loss in industrialized nations and do so as a result of abnormal retinal neovascularization. Since the retina consists of well-defined layers of neuronal, glial, and vascular elements, relatively small disturbances such as those seen in vascular proliferation or edema can lead to significant loss of visual function. Inherited retinal degenerations, such as retinitis pigmentosa (RP), are also associated with vascular abnormalities, such as arteriolar narrowing and vascular atrophy. While significant progress has been made in identifying factors that promote and inhibit angiogenesis, no treatment is currently available to specifically treat ocular vascular disease.

Inherited degenerations of the retina affect as many as 1 in 3500 individuals and are characterized by progressive night blindness, visual field loss, optic nerve atrophy, arteriolar attenuation, altered vascular permeability and central loss of vision often progressing to complete blindness (Heckenlively, J. R., editor, 1988; Retinitis Pigmentosa, Philadelphia: JB Lippincott Co.). Molecular genetic analysis of these diseases has identified mutations in over 110 different genes accounting for only a relatively small percentage of the known affected individuals (Humphries et al., 1992, Science 256:804-808; Farrar et al. 2002, EMBO J. 21:857-864.). Many of these mutations are associated with enzymatic and structural components of the phototransduction machinery including rhodopsin, cGMP phosphodiesterase, *rds* peripherin, and RPE65. Despite these observations, there are still no effective treatments to slow or reverse the progression of these retinal degenerative diseases. Recent advances in gene therapy have led to successful reversal of the *rds* (Ali et al. 2000, Nat. Genet. 25:306-310) and *rd* (Takahashi et al. 1999, J. Virol. 73:7812-7816) phenotypes in mice and the RPE65 phenotype in dogs (Acland et al. 2001, Nat. Genet. 28:92-95) when the wild type transgene is delivered to photoreceptors or the retinal pigmented epithelium (RPE) in animals with a specific mutation.

Angiogenesis is the process by which new blood vessels form. In response to specific chemical signals, capillaries sprout from existing vessels, eventually growing in size as needed by the organism. Initially, endothelial cells, which line the blood vessels, divide in a direction orthogonal to the existing vessel, forming a solid sprout. Adjacent endothelial cells then form large vacuoles and the cells rearrange so that the vacuoles orient themselves end to end and eventually merge to form the lumen of a new capillary (tube formation).

Angiogenesis is stimulated by a number of conditions, such as in response to a wound, and accompanies virtually all tissue growth in vertebrate organisms such as mammals. Angiogenesis also plays a role in certain disease states such as diabetic retinopathy and certain cancers. The growth of tumors, for example, requires blood vessel growth to provide oxygen and nutrients to the growing tumor tissue.

Angiogenesis may be arrested or inhibited by interfering with the chemical signals that stimulate the angiogenic process. For example, angiogenic endothelial cells produce proteases to digest the basal lamina that surround the blood vessels, thus clearing a path for the new capillary. Inhibition of these proteases, or their formation, can prevent new vessels from forming. Likewise, the endothelial cells proliferate in response to chemical signals. Particularly important proliferation signals include the vascular endothelial growth factor (VEGF), and the fibroblast growth factor (FGF) families of proteins. VEGF has been shown to be involved in vascularization of certain tumors. Interference with these proliferation signaling processes can also inhibit angiogenesis.

Several factors are involved in angiogenesis. Both acidic and basic fibroblast growth factor molecules are mitogens for endothelial cells and other cell types. A highly selective mitogen for vascular endothelial cells is vascular endothelial growth factor (VEGF).

In the normal adult, angiogenesis is tightly regulated, and is limited to wound healing, pregnancy and uterine cycling. Angiogenesis is turned on by specific angiogenic molecules such as basic and acidic fibroblast growth factor (FGF), VEGF, angiogenin, transforming growth factor (TGF), tumor necrosis factor-α (TNF-α) and platelet derived growth factor (PDGF). Angiogenesis can be suppressed by inhibitory molecules such as interferon-α, thrombospondin-1, angiostatin and endostatin. It is the balance of these naturally occurring stimulators and inhibitors that controls the normally quiescent capillary vasculature. When this balance is upset, as in certain disease states, capillary endothelial cells are induced to proliferate, migrate and ultimately differentiate.

Angiogenesis plays a central role in a variety of disease including cancer and ocular neovascularization. Sustained growth and metastasis of a variety of tumors has also been shown to be dependent on the growth of new host blood vessels into the tumor in response to tumor derived angiogenic factors. Proliferation of new blood vessels in response to a variety of stimuli occurs as the dominant finding in the majority of eye disease and that blind including proliferative diabetic retinopathy, ARMD, rubeotic glaucoma, interstitial keratitis and retinopathy of prematurity. In these diseases, tissue damage can stimulate release of angiogenic factors resulting in capillary proliferation. VEGF plays a dominant role in iris neovascularization and neovascular retinopathies. While reports clearly show a correlation between intraocular VEGF levels and ischemic retinopathic ocular neovascularization, FGF likely plays a role. Basic and acidic FGF are known to be present in the normal adult retina, even though detectable levels are not consistently correlated with neovascularization. This may be largely due to the fact that FGF binds very tightly to charged components of the extracellular matrix and may not be readily available in a freely diffusible form that would be detected by standard assays of intraocular fluids.

A final common pathway in the angiogenic response involves integrin-mediated information exchange between a proliferating vascular endothelial cell and the extracellular matrix. This class of adhesion receptors, called integrins, are expressed as heterodimers having an α and β subunit on all cells. One such integrin, αᵥβ₃, is the most promiscuous member of this family and allows endothelial cells to interact with a wide variety of extracellular matrix components. Peptide and antibody antagonists of this integrin inhibit angiogenesis by selectively inducing apoptosis of the proliferating vascular endothelial cells. Two cytokine-dependent pathways of angiogenesis exist and may be defined by their dependency on distinct vascular cell integrins, αᵥβ₃ and αᵥβ₅. Specifically, basic FGF- and VEGF-induced angiogenesis depend on integrin αᵥβ₃ and αᵥβ₅, respectively, since antibody antagonists of each integrin selectively block one of these angiogenic pathways in the rabbit corneal and chick chorioallantoic membrane (CAM) models. Peptide antagonists that block all αᵥ integrins inhibit FGF- and VEGF-stimulated angiogenesis. While normal human ocular blood vessels do not display either integrin, αᵥβ₃ and αᵥβ₅ integrins are selectively displayed on blood vessels in tissues from patients with active neovascular eye disease. While only αᵥβ₃ was consistently observed in tissue from patients with ARMD, αᵥβ₃ and αᵥβ₅ both were present in tissues from patients with proliferative diabetic retinopathy. Systemically administered peptide antagonists of integrins blocked new blood vessel formation in a mouse model of retinal vasculogenesis.

Testing potential treatments for retinal neovascular diseases has been greatly facilitated by the development of models of oxygen-induced retinopathy (OIR) in several animal species, including the kitten, the beagle puppy, the rat, and the mouse. In each of these models, exposing new-born animals to hyperoxia (or to alternating hyperoxia and hypoxia) prompts regression or delay of retinal vascular development, followed by abnormal angiogenesis after their return to normal oxygen levels. These models mirror the events that occur during retinopathy of prematurity (ROP), a condition involving pathological neovascularization that can affect premature infants.

Over the last decade, the mouse model of OIR has become the most common model for studying abnormal angiogenesis associated with oxygen-induced retinopathies. The pattern of vascular abnormalities in this model differs slightly from that observed in ROP; in the mouse the central, posterior retina becomes avascular following exposure to hyperoxia while in human infants the periphery is avascular. Nonetheless, this is a well-accepted model for studying disease mechanisms and potential treatment of hypoxia-induced retinopathy, and the vascular changes are very consistent, reproducible and quantifiable. In recent years, the use of this model has been extended to the general study of ischemic vasculopathies and related anti-angiogenic interventions, and it is now used extensively in both basic and applied research environments.

The historically common method for quantifying the proliferative neovascular response in the mouse OIR model is based upon counting the number of cells associated with neovessels extending from the retina into the vitreous ("pre-inner limiting membrane (ILM) nuclei"). This is done in sagittal cross sections, usually in regions near (but not including) the optic disk. The method is very labor-intensive, time consuming, and is fraught with difficulties, including the need to differentiate cells of the abnormal vessels from those of the hyaloidal vessels in the vitreous. Because, in general, only every 30th serial section is evaluated, a large portion of the tissue is not quantified potentially introducing large sampling errors. In addition, as whole eyes are immediately sectioned, it prevents same-eye assessment of another important parameter of this model, namely the extent of vascular obliteration and rate of retinal revascularization which occurs concomitantly with abnormal neovascular tufts formation. This parameter is best assessed in retinal whole retinal mount preparations.

For many years it has been known that a population of stem cells exists in the normal adult circulation and bone marrow. Different sub-populations of these cells can differentiate along hematopoietic lineage positive (Lin⁺) or lineage negative (Lin⁻) lineages. Furthermore, the lineage negative hematopoietic stem cell (HSC) population has recently been shown to contain endothelial progenitor cells (EPC) capable of forming blood vessels *in vitro* and *in vivo* (*See* Asahara et al. 1997, Science 275: 964-7). These cells can participate in normal and pathological postnatal angiogenesis (*See* Lyden et al. 2001 Nat. Med. 7, 1194-201; Kalka et al. 2000, Proc. Natl. Acad. Sci. U. S. A. 97:3422-7; and Kocher et al. 2001, Nat. Med. 7: 430-6) as well as differentiate into a variety of non-endothelial cell types including hepatocytes (*See* Lagasse et al. 2000, Nat. Med. 6:1229-34), microglia *(See* Priller et al. 2002 Nat. Med. 7:1356-61), cardiomyocytes (*See* Orlic et al. 2001, Proc. Natl. Acad Sci. U. S. A. 98:10344-9) and epithelium *(See* Lyden et al. 2001, Nat. Med. 7:1194-1201). Although these cells have been used in several experimental models of angiogenesis, the mechanism of EPC targeting to neovasculature is not known and no strategy has been identified that will effectively increase the number of cells that contribute to a particular vasculature.

Hematopoietic stem cells from bone marrow are currently the only type of stem cell commonly used for therapeutic applications. Bone marrow HSC's have been used in transplants for over 40 years. Currently, advanced methods of harvesting purified stem cells are being investigated to develop therapies for treatment of leukemia, lymphoma, and inherited blood disorders. Clinical applications of stem cells in humans have been investigated for the treatment of diabetes and advanced kidney cancer in limited numbers of human patients.

WO2004/010959 discloses the use of lineage-negative haematopoietic stem cells (Lin-HSC) derived from bone marrow and transfected with angiostatic fragments of TrpRS, for preparing a medication against retinopathy.

### SUMMARY OF THE INVENTION

The present invention provides a use of cells in the preparation of a pharmaceutical composition for treating a mammal at risk of developing or suffering from a retinopathic disease, in particular a retinopathy of prematurity, wherein:
(a) the cells are an isolated, lineage-negative, vasculotropic, myeloid-like bone marrow cell population in which the cells express the CD44 and CD11b antigens;
(b) the cells have been transfected with a nucleic acid encoding an angiostatic fragment of tryptophanyl-tRNA synthetase (Trp-RS);
(c) the pharmaceutical composition is for administration of said cells to the retina of the mammal in an amount effective to promote physiological revascularization of damaged areas of the retina and to ameliorate damage to the retina caused by the disease.

Preferably, the mammal is a human patient. In one preferred embodiment, the cells comprise hematopoietic stem cells and endothelial progenitor cells.

Preferably, the cells are autologous to the individual mammal being treated. Preferably, the pharmaceutical composition is for administration of the cells by intraocular injection. In a preferred embodiment, the pharmaceutical composition is for administration of the cells to a mammal suffering from retinopathy of prematurity (ROP), such as a human infant, during early stages of the disease. In another preferred embodiment, the pharmaceutical composition is for administration of the cells administered to a mammal at risk of developing ROP or a related retinopathic condition, as a prophylactic agent, prior to the onset of disease symptoms of prior to exposure of an infant to hyperoxia.

Results from the oxygen induced retinopathy (OIR) model of ROP indicate that the treatment described herein promotes healing and vascular recovery in the retina of a mammal that suffers from the retinopathic disease. In addition, the treatment promotes recovery of visual neurons due to neurotrophic effects of the cells. Beneficially, the cells used according to the invention incorporate into the vasculature of the retina and differentiate into endothelial cells, while at the same time incorporating into the neuronal network and ameliorating the degeneration of neuronal cells, such as cone cells in the retina. The cells include cells that selectively target activated retinal astrocytes when intravitreally injected into the eye, and remain stably incorporated into neovasculature and neuronal network of the eye.

A particular advantage of ocular treatments described herein is a vasculotrophic and neurotrophic rescue effect observed in eyes intravitreally treated with the cells used according to the invention . Retinal neurons and photoreceptors, particularly cones, are preserved and some measure of visual function can be maintained in eyes treated with the cells.

Preferably, the diseased retina to be treated according to the invention includes activated astrocytes. This can be accomplished by early treatment of the eye with the cells used according to the invention when there is an associated gliosis, or by using a laser to stimulate local proliferation of activated astrocytes.

### BRIEF DESCRIPTION OF THE DRAWINGS

In the DRAWINGS:

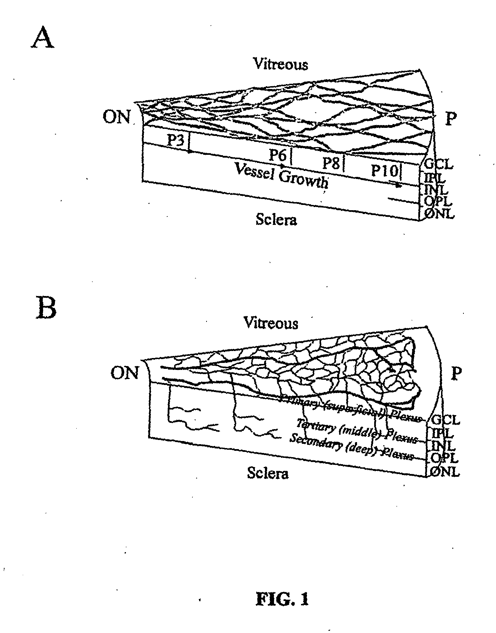

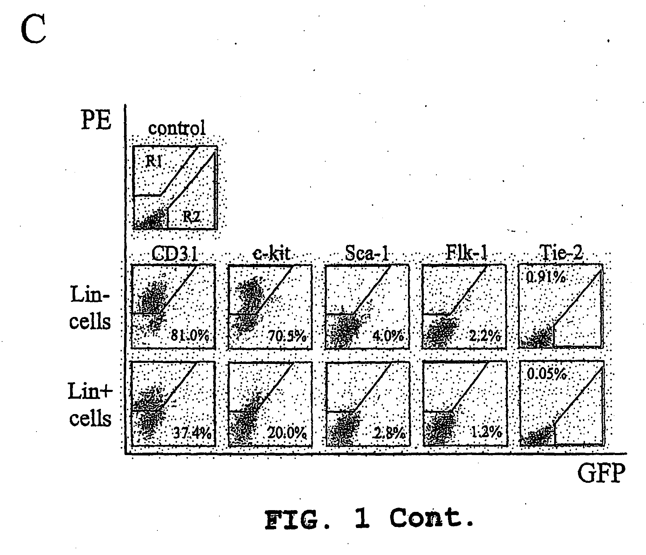

FIG. 1 depicts schematic diagrams of developing mouse retina. (**a**) Development of primary plexus. (**b**) The second phase of retinal vessel formation. GCL, ganglion cell layer; IPL, inner plexus layer; INL, inner nuclear layer; OPL, outer plexus layer; ONL, outer nuclear layer; RPE, retinal pigment epithelium; ON, optic nerve; P, periphery. Panel (**c**) depicts flow cytometric characterization of bone marrow-derived Lin⁺ HSC and Lin⁻HSC separated cells. Top row: Dot plot distribution of non-antibody labeled cells, in which R1 defines the quantifiable-gated area of positive PE-staining; R2 indicates GFP-positive; Middle row: Lin⁻HSC (C57B/6) and Bottom row: Lin⁺ HSC (C57B/6) cells, each cell line labeled with the PE-conjugated antibodies for Sca-1, c-kit, Flk-1/KDR, CD31. Tie-2 data was obtained from Tie-2-GFP mice. Percentages indicate percent of positive-labeled cells out of total Lin⁻HSC or Lin⁺ HSC population.

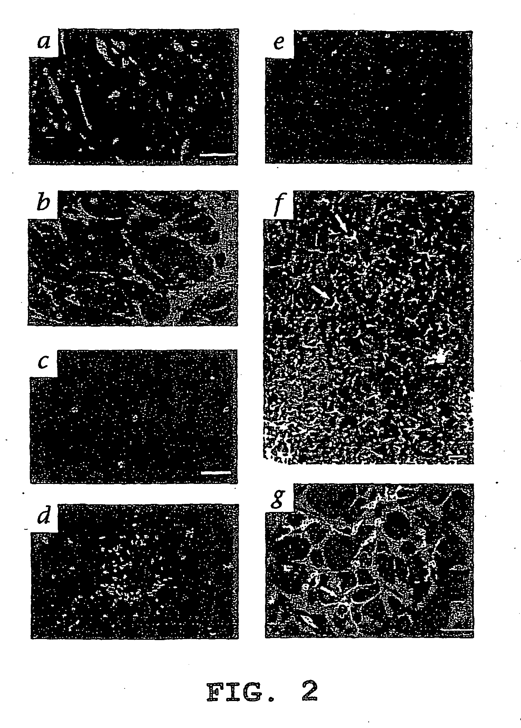

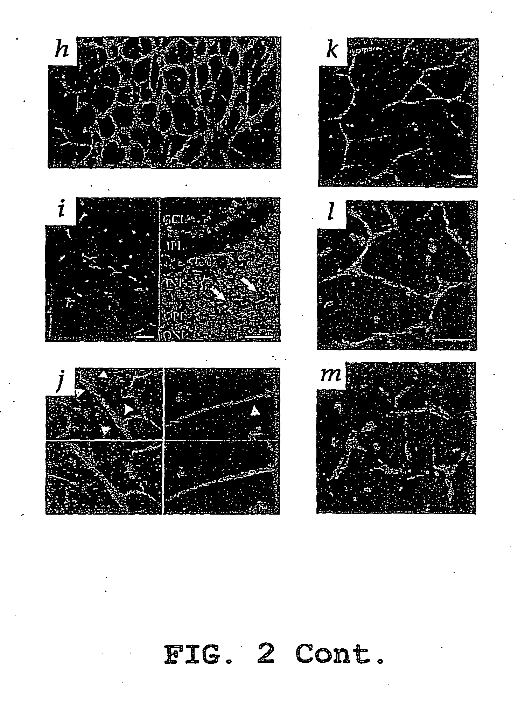

FIG. 2 depicts engraftment of Lin⁻HSCs into developing mouse retina. (**a**) At four days post-injection (P6) intravitreally injected eGFP⁺ Lin⁻HSC cells attach and differentiate on the retina. (**b**) Lin⁻HSC (B6.129S7-Gtrosa26 mice, stained with β-gal antibody) establish themselves ahead of the vasculature stained with collagen IV antibody (asterisk indicates tip of vasculature). (**c**) Most of Lin⁺HSC cells (eGFP⁺) at four days post-injection (P6) were unable to differentiate. (**d**) Mesenteric eGFP⁺ murine EC four days post-injection (P6). (**e**) Lin⁻ HSCs (eGFP⁺) injected into adult mouse eyes. (**f**) Low magnification of eGFP⁺ Lin⁻ HSCs (arrows) homing to and differentiating along the pre-existing astrocytic template in the GFAP-GFP transgenic mouse. (g) Higher magnification of association between Lin⁻ cells (eGFP) and underlying astrocyte (arrows). (h) Non-injected GFAP-GFP transgenic control. (i) Four days post-injection (P6), eGFP⁺ Lin⁻HSCs migrate to and undergo differentiation in the area of the future deep plexus. Left figure captures Lin⁻ HSC activity in a whole mounted retina; right figure indicates location of the Lin⁻ cells (arrows) in the retina (top is vitreal side, bottom is scleral side). (**j**) Double labeling with α-CD31-PE and α-GFP-alexa 488 antibodies. Seven days after injection, the injected Lin⁻HSCs (eGFP, red) were incorporated into the vasculature (CD31). Arrowheads indicate the incorporated areas. (**k**) eGFP⁺ Lin⁻ HSC cells form vessels fourteen days post-injection (P17). (**l** and **m**) Intra-cardiac injection of rhodamine-dextran indicates that the vessels are intact and functional in both the primary (I) and deep plexus (**m**).

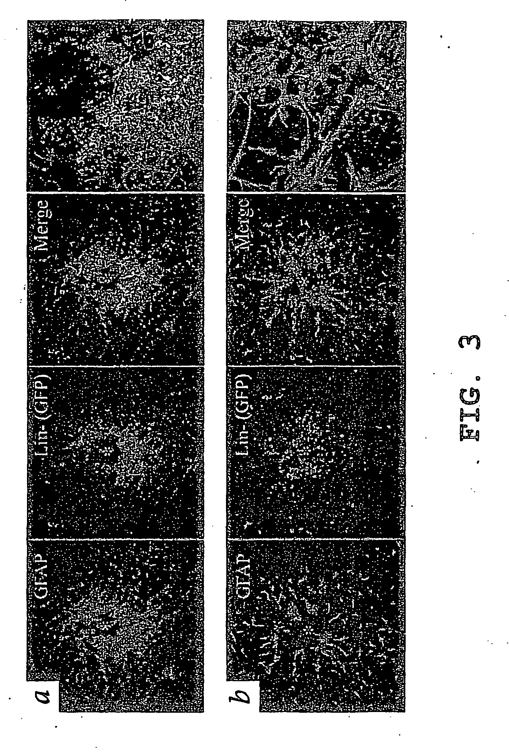

FIG. 3 shows that eGFP⁺ Lin⁻HSC cells home to the gliosis (indicated by GFAP expressing-astrocytes, far left image) induced by both laser (**a**) and mechanical (**b**) induced injury in the adult retina (asterisk indicates injured site). Far right images are a higher magnification, demonstrating the close association of the Lin⁻HSCs and astrocytes. Calibration bar= 20µM.

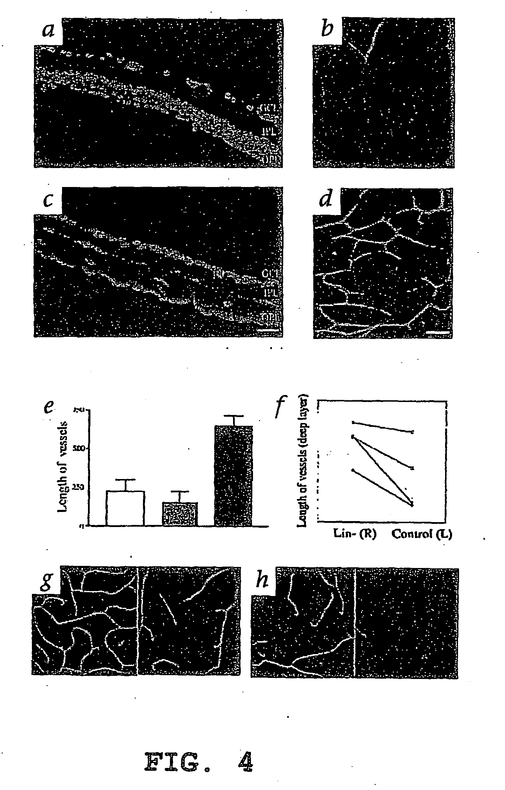

FIG. 4 shows that Lin⁻HSC cells rescue the vasculature of the retinal degeneration mouse. (**a-d**) Retinas at 27 days post-injection (P33) with collagen IV staining; (**a**) and (**b**), retinas injected with Lin⁺ HSC cells (Balb/c) showed no difference in vasculature from normal FVB mice; (**c**) and (**d**) retinas injected with Lin⁻HSCs (Balb/c) exhibited a rich vascular network analogous to a wild-type mouse; (**a**) and (c), frozen sections of whole retina (top is vitreal side, bottom is scleral side) with DAPI staining; (**b**) and (**d**), deep plexus of retinal whole amount; (**e**) bar graph illustrating the increase in vascularity of the deep vascular plexus formed in the Lin⁻HSC cell-injected retinas (n=6). The extent of deep retinal vascularization was quantified by calculating the total length of vessels within each image. Average total length of vessels/high power field (in microns) for Lin⁻HSC, Lin⁺HSC or control retinas were compared. (**f**) Comparison of the length of deep vascular plexus after injection with Lin⁻HSC (R, right eye) or Lin⁺HSC (L, left eye) cells from *rd*/*rd* mouse. The results of six independent mice are shown (each color represents a separate mouse). (g) and (**h**) Lin⁻HSC cells also (Balb/c) rescued the *rd*/*rd* vasculature when injected into P15 eyes. The intermediate and deep vascular plexus of Lin⁻HSC (G) or Lin⁺HSC (H) cell injected retinas (one month after injection) are shown.

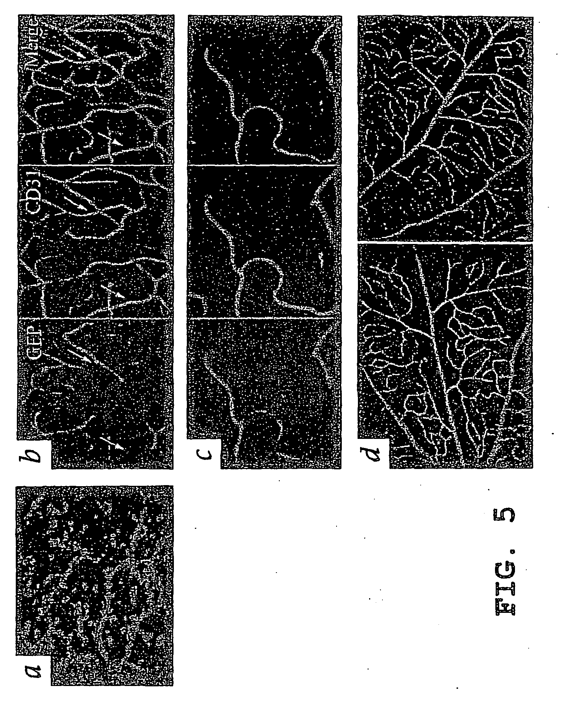

FIG. 5 depicts photomicrographs of mouse retinal tissue: (**a**) deep layer of retinal whole mount (*rd*/*rd* mouse), five days post-injection (P11) with eGFP⁺ Lin⁻HSCs visible (gray). (**b**) and (**c**) P60 retinal vasculature of Tie-2-GFP (*rd*/*rd*) mice that received Balb/c Lin⁻ cells (b) or Lin⁺HSC cell (**c**) injection at P6. Only endogenous endothelial cells (GFP-stained) are visible in the left panels of (**b**) and (**c**). The middle panels of (**b**) and (**c**) are stained with CD31 antibody; arrows indicate the vessels stained with CD31 but not with GFP, the right panels of (b) and (c) show staining with both GFP and CD31. (d) α-SMA staining of Lin⁻ HSC injected (left panel) and control retina (right panel).

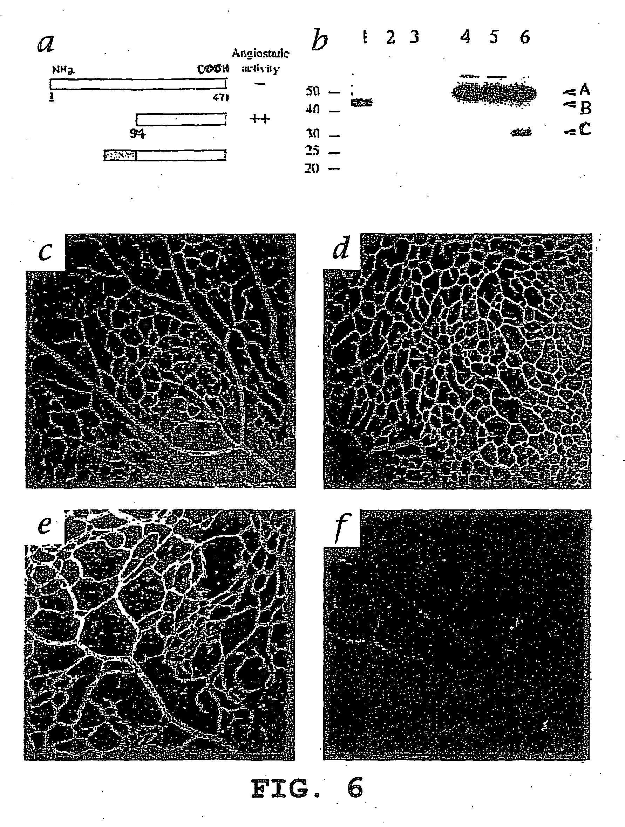

FIG. 6 shows that T2-TrpRS-transfected Lin⁻ HSCs inhibit the development of mouse retinal vasculature. (**a**) Schematic representation of human TrpRS, T2-TrpRS and T2-TrpRS with an Igk signal sequence at the amino terminus. (**b**) T2-TrpRS transfected Lin⁻ HSC-injected retinas express T2-TrpRS protein *in vivo.* (1) Recombinant T2-TrpRS produced in *E. coli;* (2) Recombinant T2-TrpRS produced in *E. coli;* (3) Recombinant T2-TrpRS produced in *E*. *coli*; (4) control retina; (5) Lin⁻HSC + pSecTag2A (vector only) injected retina; (6) Lin⁻HSC + pKLe135 (Igk-T2-TrpRS in pSecTag) injected retina. (A) Endogenous TrpRS. (B) Recombinant T2-TrpRS. (C) T2-TrpRS of Lin⁻HSC injected retina. (c-f) Representative primary (superficial) and secondary (deep) plexuses of injected retinas, seven days post-injection; (**c**) and (**d**) Eyes injected with empty plasmid-transfected Lin⁻HSC developed normally; (**e**) and (**f**) the majority of T2-TrpRS-transfected Lin⁻HSC injected eyes exhibited inhibition of deep plexus; (**c**) and (**e**) primary (superficial) plexus; (**d**) and (**f**) secondary (deep) plexus). Faint outline of vessels observed in (**f**) are "bleed-through" images of primary network vessels shown in (**e**).

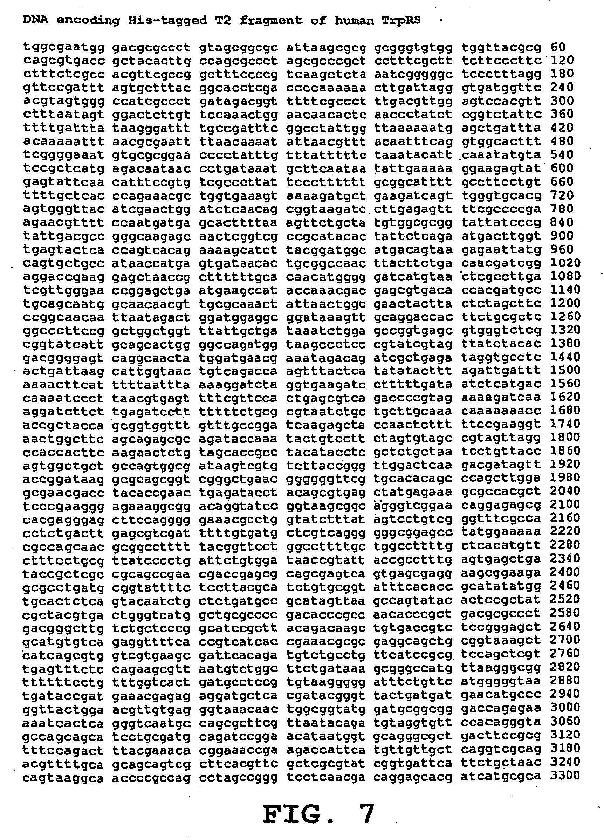

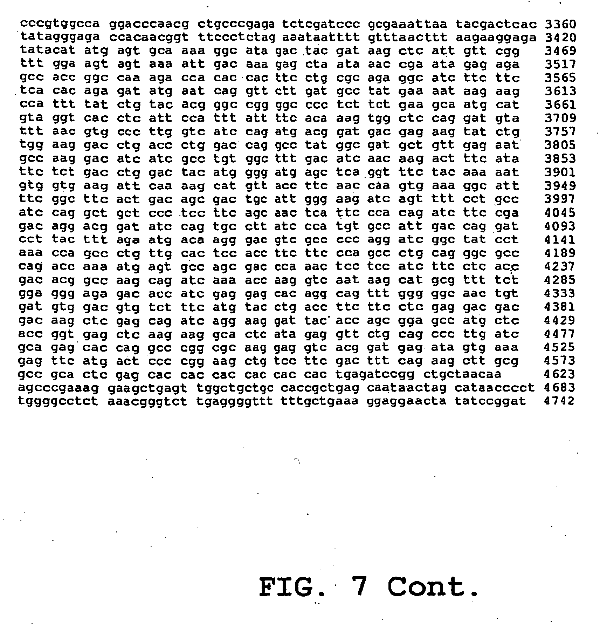

FIG. 7 shows the DNA sequence encoding His₆-tagged T2-TrpRS, SEQ ID NO: 1.

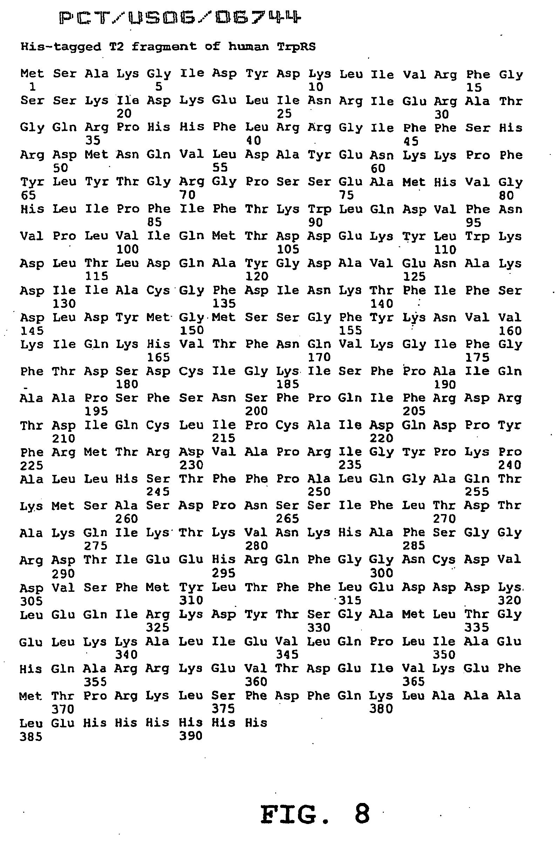

FIG. 8 shows the amino acid sequence of His₆-tagged T2-TrpRS, SEQ ID NO: 2.

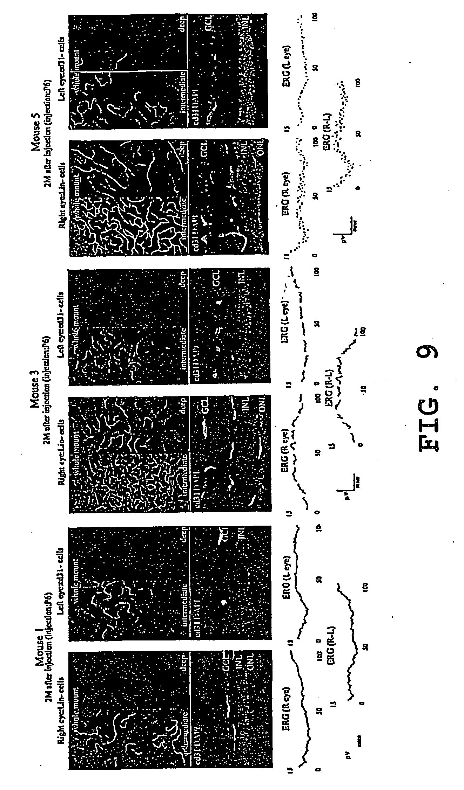

FIG. 9 illustrates photomicrographs and electroretinograms (ERG) of retinas from mice whose eyes were injected with the Lin⁻HSC and with Lin⁺HSC (controls).

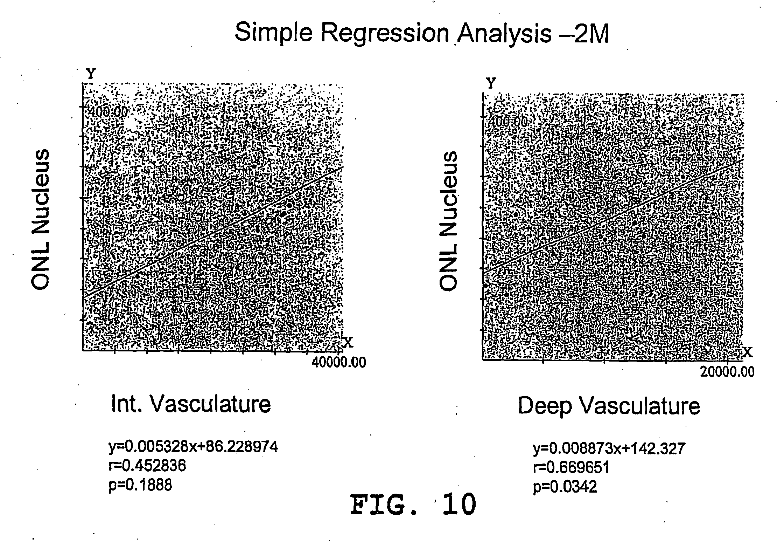

FIG. 10 depicts statistical plots showing a correlation between neuronal rescue (y-axis) and vascular rescue (x-axis) for both the intermediate (Int.) and deep vascular layers of *rd*/*rd* mouse eyes treated with Lin⁻HSC.

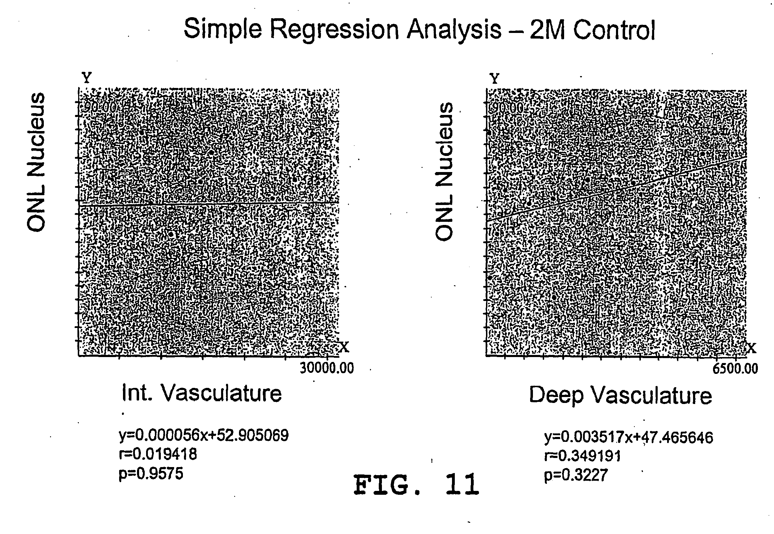

FIG. 11 depicts statistical plots showing no correlation between neuronal rescue (y-axis) and vascular rescue (x-axis) for *rd*/*rd* mouse eyes that were treated with Lin⁺HSC.

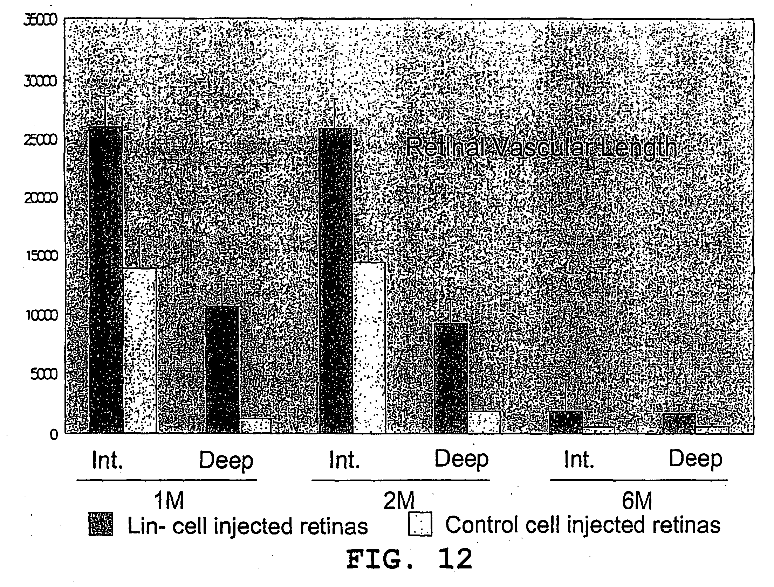

FIG. 12 is a bar graph of vascular length (y-axis) in arbitrary relative units for rd/rd mouse eyes treated with the Lin⁻ HSC (dark bars) and untreated (light bars) *rd*/*rd* mouse eyes at time points of 1 month (1M), 2 months (2M), and 6 months (6M) post-injection.

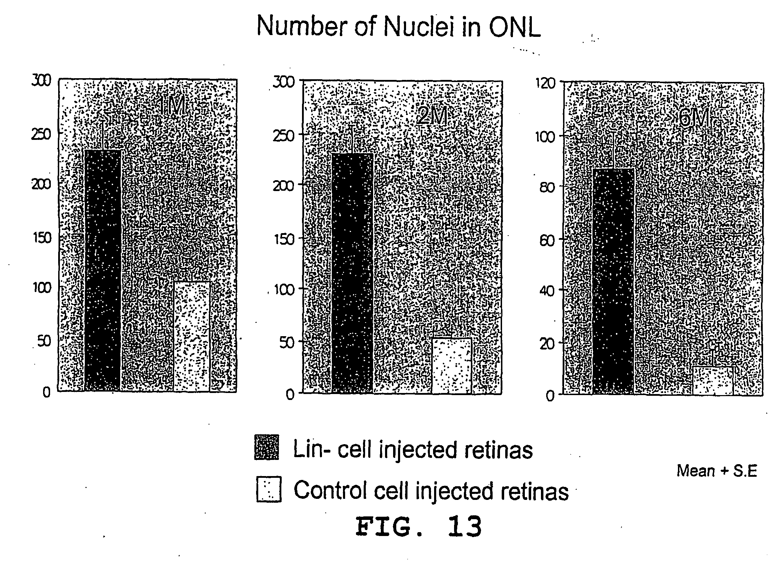

FIG. 13 includes three bar graphs of the number of nuclei in the outer neural layer (ONR) of *rd*/*rd* mice at 1 month (1M), 2 months (2M) and 6 months (6M), post-injection, and demonstrates a significant increase in the number of nuclei for eyes treated with Lin⁻HSC (dark bars) relative to control eyes treated with Lin⁺ HSC (light bars).

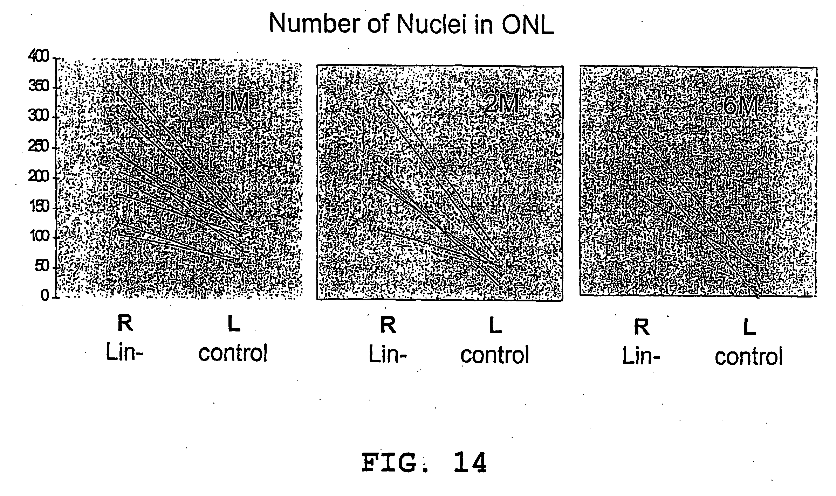

FIG. 14 depicts plots of the number of nuclei in the outer neural layer for individual *rd*/*rd* mice, comparing the right eye (R, treated with Lin⁻ HSC) relative to the left eye (L, control eye treated with Lin⁺ HSC) at time points (post injection) of 1 month (1M), 2 months (2M), and 6 months (6M); each line in a given plot compares the eyes of an individual mouse.

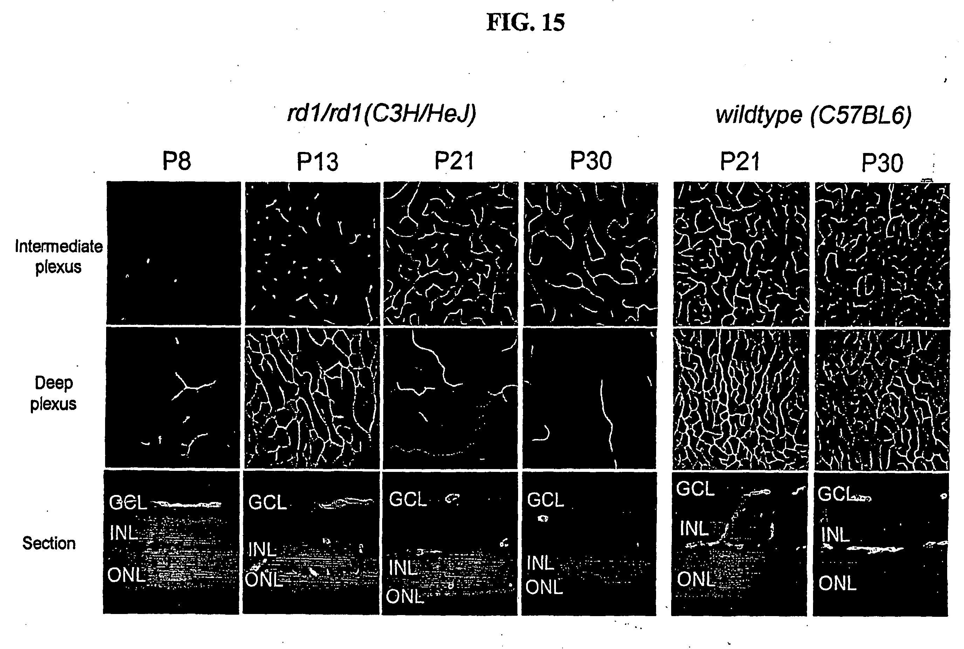

FIG. 15 depicts retinal vasculature and neural cell changes in *rd1*/*rd1* (C3H/HeJ, left panels) or wild type mice (C57BL/6, right panels). Retinal vasculature of intermediate (upper panels) or deep (middle panels) vascular plexuses in whole-mounted retinas (red: collagen IV, green: CD31) and sections (red: DAPI, green: CD31, lower panels) of the same retinas are shown (P: postnatal day). (GCL: ganglion cell layer, INL: inter nuclear layer, ONL: outer nuclear layer).

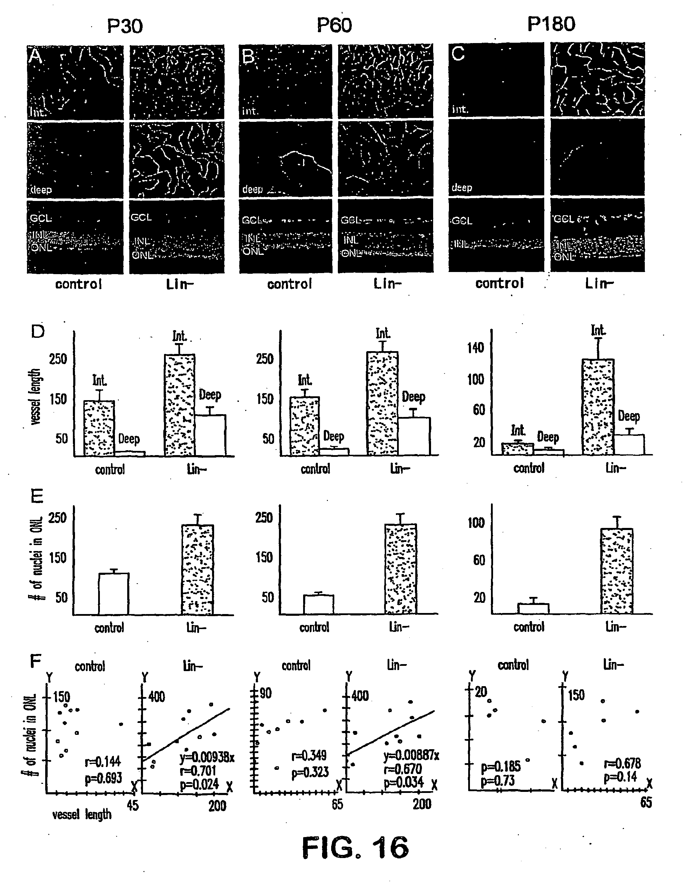

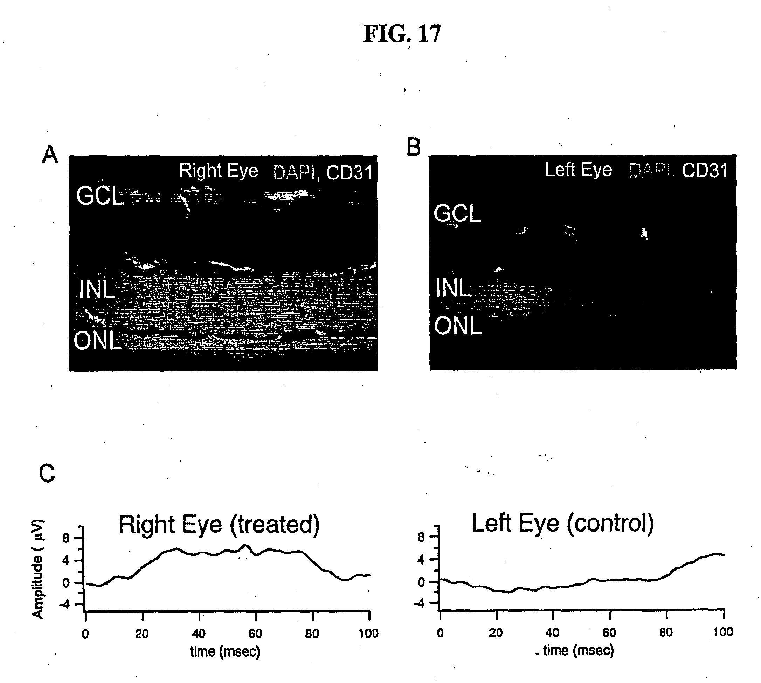

FIG. 16 shows that Lin⁻HSC injection rescues the degeneration of neural cells in *rd1*/*rd1* mice. (A, B and C), retinal vasculature of intermediate (Int.) or deep plexus and sections of Lin⁻HSC injected eye (right panels) and contralateral control cell (CD31⁻) injected eye (left panels) at P30 (**A**), P60 (**B**), and P180 (**C**). (**D**), the average total length of vasculature (+ or - standard error of the mean) in Lin⁻HSC injected or control cell (CD31⁻) injected retinas at P30 (left, n=10), P60 (middle, n=10), and P180 (right, n=6). Data of intermediate (Int.) and deep vascular plexus are shown separately (Y axis: relative length of vasculature). (**E**), the average numbers of cell nuclei in the ONL at P30 (left, n=10), P60 (middle, n=10), or P180 (right, n=6) of control cell (CD31⁻) or Lin⁻HSC injected retinas (Y axis: relative number of cell nuclei in the ONL). (**F**), Linear correlations between the length of vasculature (X axis) and the number of cell nuclei in the ONL (Y axis) at P30 (left), P60 (middle), and P180 (right) of Lin⁻HSC or control cell injected retinas. FIG. 17 demonstrates that retinal function is rescued by Lin⁻HSC injection. Electroretinographic (ERG) recordings were used to measure the function of Lin⁻HSC or control cell (CD31⁻) injected retinas. (**A** and **B**), Representative cases of rescued and non-rescued retinas 2 months after injection. Retinal section of Lin⁻ HSC injected right eye (**A**) and CD31⁻ control cell injected left eye (**B**) of the same animal are shown (green: CD31 stained vasculature, red: DAPI stained nuclei). (**C**), ERG results from the same animal shown in (**A**) and (**B**).

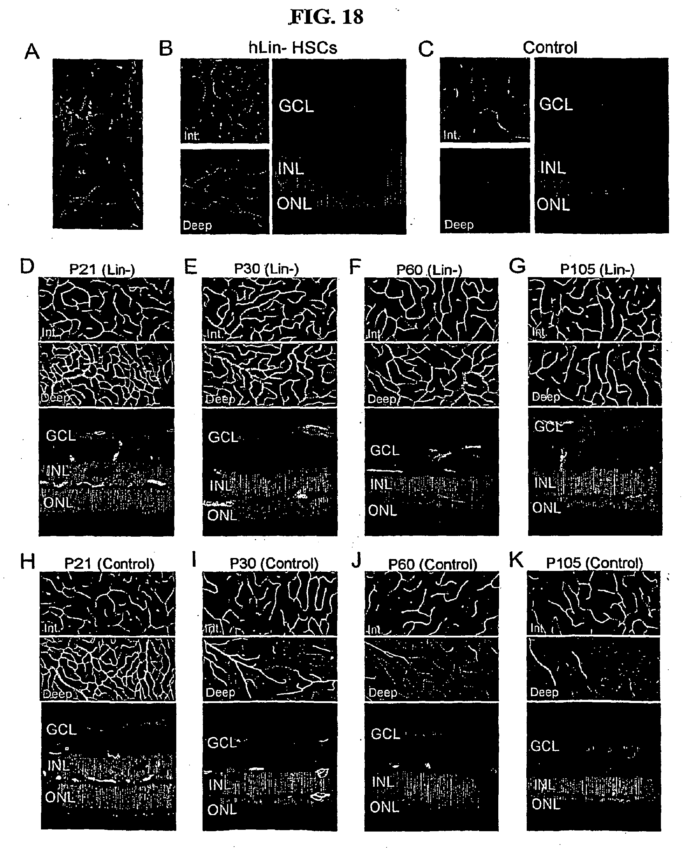

FIG. 18 shows that a population of human bone marrow cells can rescue degenerating retinas in the *rd1* mouse (**A-C**). The rescue is also observed in another model of retinal degeneration, *rd10* (**D-K**). **A**, human Lin⁻HSCs (hLin⁻HSCs) labeled with green dye can differentiate into retinal vascular cells after intravitreal injection into C3SnSmn.CB17-*Prkdc SCID* mice. (**B** and **C**), Retinal vasculature (left panels; upper: intermediate plexus, lower: deep plexus) and neural cells (right panel) in hLin⁻HSC injected eye (**B**) or contralateral control eye (**C**) 1.5 months after injection. (**D-K**), Rescue of *rd10* mice by Lin⁻HSCs (injected at P6). Representative retinas at P21 (**D**: Lin⁻HSCs, **H**: control cells), P30 (**E**: Lin⁻HSCs, **I**: control cells), P60 (**F**: Lin⁻HSCs, **J**: control cells), and P105 (**G**: Lin⁻HSCs, **K**: control cells) are shown (treated and control eyes are from the same animal at each time point). Retinal vasculature (upper image in each panel is the intermediate plexus; the middle image in each panel is the deep plexus) was stained with CD31 (green) and Collagen IV (red). The lower image in each panel shows a cross section made from the same retina (red: DAPI, green: CD31).

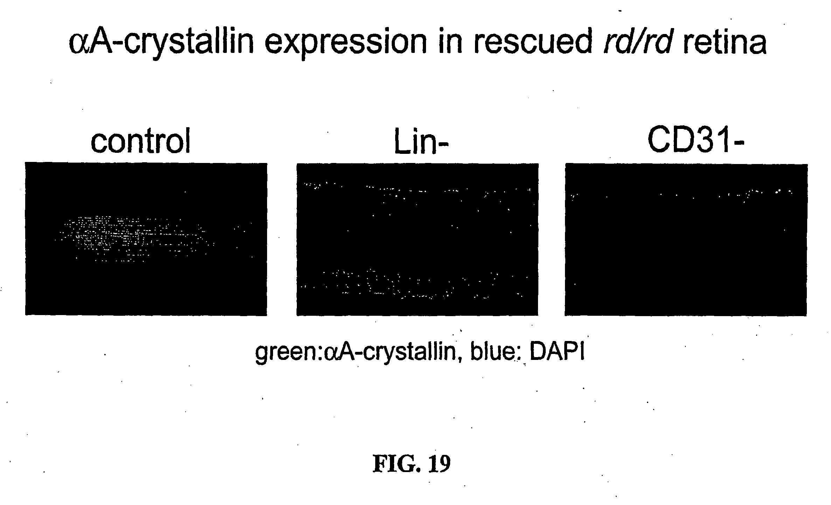

FIG. 19 demonstrates that crystallin αA is up regulated in rescued outer nuclear layer cells after treatment with Lin⁻HSCs but not in contralateral eyes treated with control cells. Left panel; IgG control in rescued retina, Middle panel; crystallin αA in rescued retina, Right panel; crystallin αA in non-rescued retina.

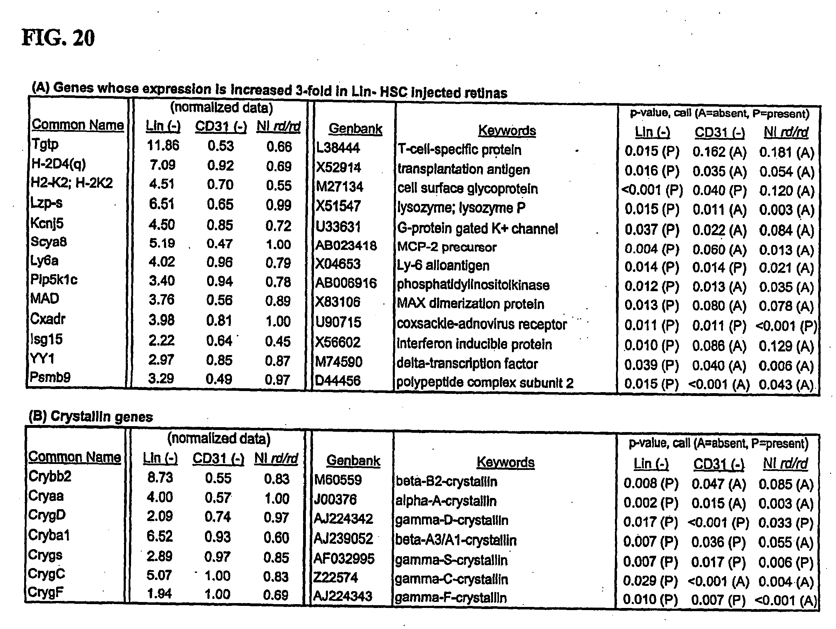

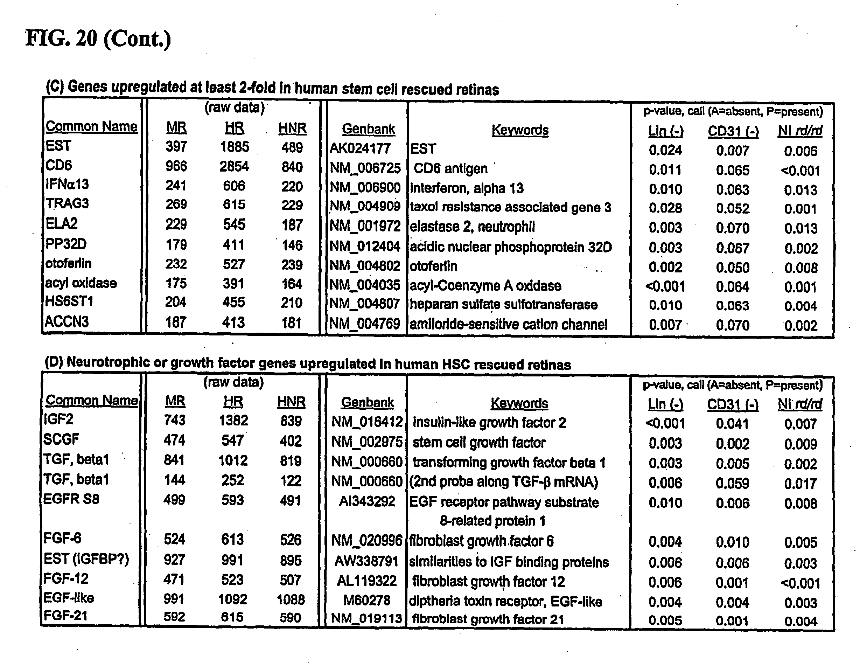

FIG. 20 includes tables of genes that are upregulated in murine retinas that have been treated with Lin⁻ HSCs. (**A**) Genes whose expression is increased 3-fold in mouse retinas treated with murine Lin⁻ HSCs. (**B**) Crystallin genes that are upregulated in mouse retinas treated with murine Lin⁻ HSC. (C) Genes whose expression is increased 2-fold in mouse retinas treated with human Lin⁻ HSCs. (**D**) Genes for neurotrophic factors or growth factors whose expression is upregulated in mouse retinas treated with human Lin⁻ HSCs.

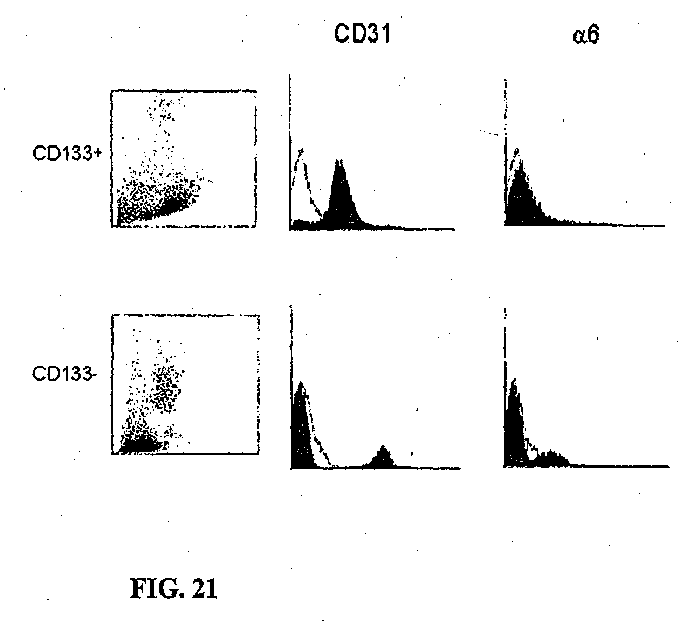

FIG. 21 illustrates the distribution of CD31 and integrin α6 surface antigens on CD133 positive (DC133⁺) and CD133 negative (CD133⁻) human Lin⁻ HSC populations. The left panels show flow cytometry scatter plots. The center and right panels are histograms showing the level of specific antibody expression on the cell population. The Y axis represents the number of events and the X axis shows the intensity of the signal. A filled histogram shifted to the right of the outlined (control) histogram represents an increased fluorescent signal and expression of the antibody above background level.

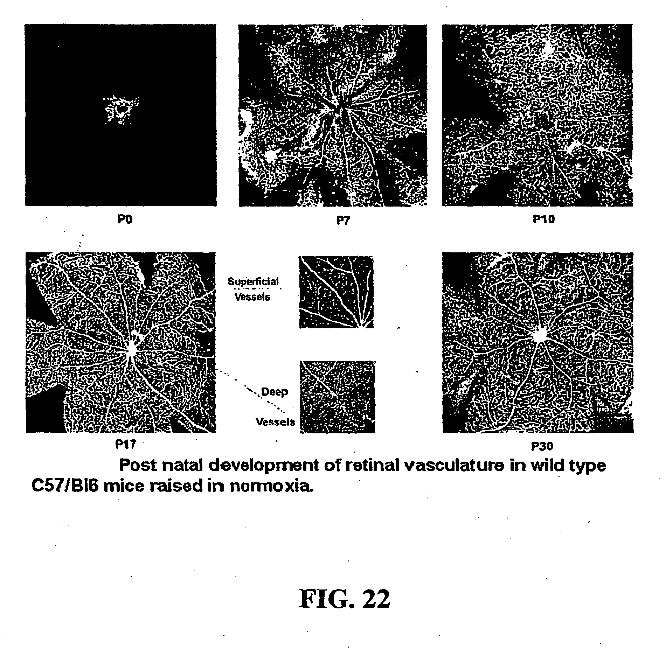

FIG. 22 illustrates postnatal retinal development in wild-type C57/B16 mice raised in normal oxygen levels (normoxia), at post natal days P0 through P30.

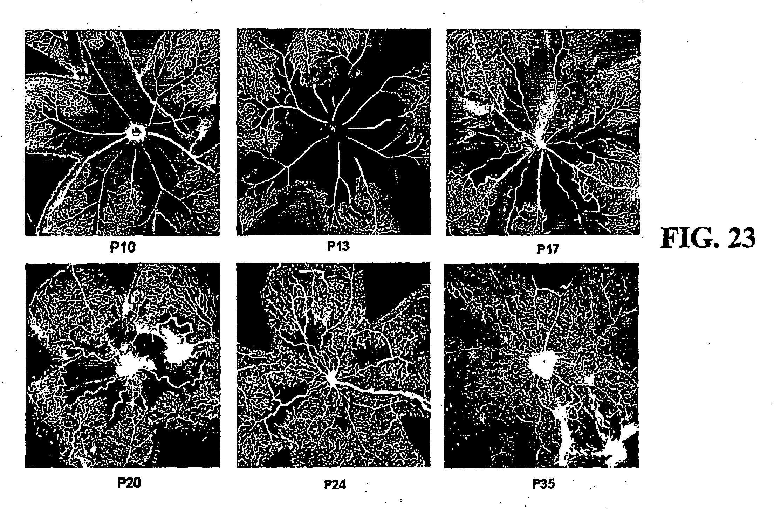

FIG. 23 illustrates oxygen-induced retinopathy model in C57/B16 mice raised in high oxygen levels (hyperoxia; 75% oxygen) between P7 and P12, followed by normoxia from P12-P17.

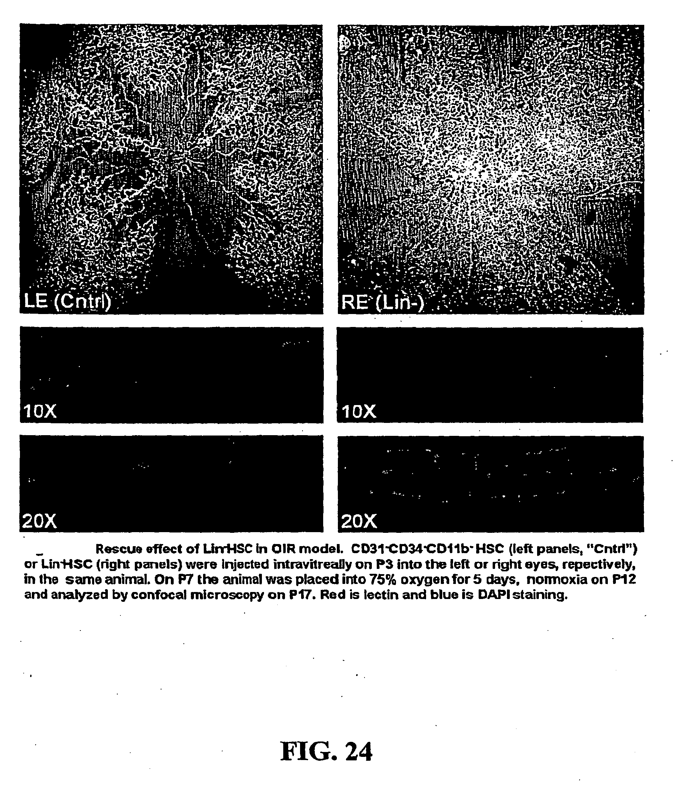

FIG. 24 demonstrates vascular rescue by treatment with the Lin⁻ HSC populations in the oxygen-induced retinopathy (OIR) model.

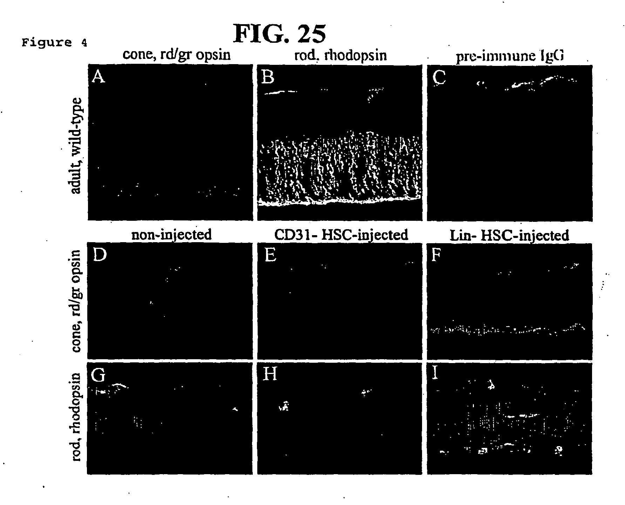

FIG. 25 shows rescued photoreceptors in *rd1* mouse outer nuclear layer (ONL) following intravitreal injection of Lin-HSC are predominantly cones. A small percentage of photoreceptors in the wild type mouse retina (upper panel) were cones as evidenced by expression of red/green cone opsin (**A**) while most cells of the ONL were positive for rod specific rhodopsin (**B**). Retinal vasculature autofluoresces with pre-immune serum (**C**) but nuclear layers were completely negative for staining with rod or cone-specific opsins. *Rd*/*rd* mouse retinas (lower panels) had a diminished inner nuclear layer and a nearly completely atrophic ONL, both of which were negative for cone (**D**) or rod (Panel **G**) opsin. Control, CD31- HSC treated eyes are identical to non-injected *rd*/*rd* retinas, without any staining for cone (**E**) or rod (**H**) opsin. Lin-HSC treated contralateral eyes exhibited a markedly reduced, but clearly present ONL that is predominantly comprised of cones, as evidenced by positive immunoreactivity for cone red/green opsin (**F**). A small number of rods were also observed (**I**).

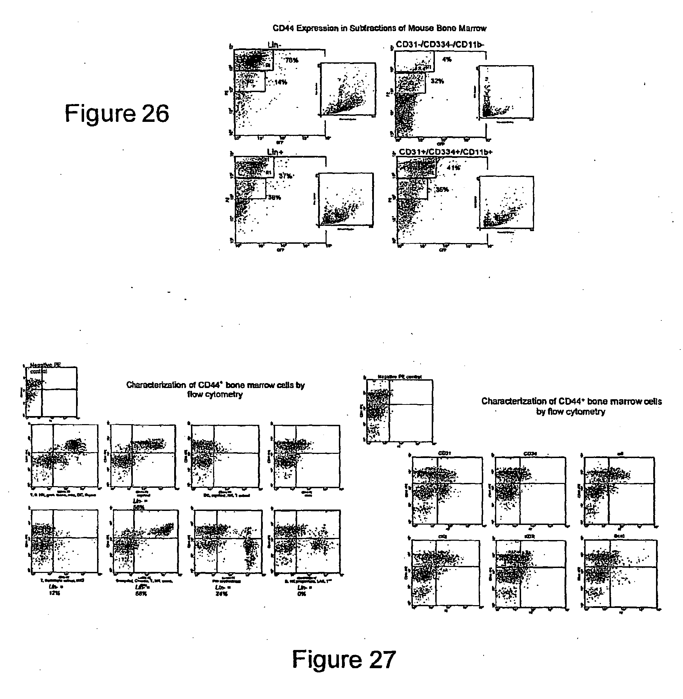

FIG. 26 shows scatter plots from flow cytometry characterization of lineage negative and lineage positive stem cell populations (upper left and lower left plots, respectively) showing percentages of cells that express the CD44 antigen (data points in red); as well as plots of CD31 negative and CD31 positive cell populations (upper right and lower right plots, respectively), showing percentages of cells that express the CD44 antigen (data points in red).
FIG. 27 shows scatter plots from flow cytometry characterization of a lineage negative cell population that expresses a significant level of CD44 antigen (left set of plots) and a sub-population of bone marrow cells that do not express a significant level of CD44 antigen (right set of plots) illustrating the relative percentages of cells expressing various other cell surface antigens.

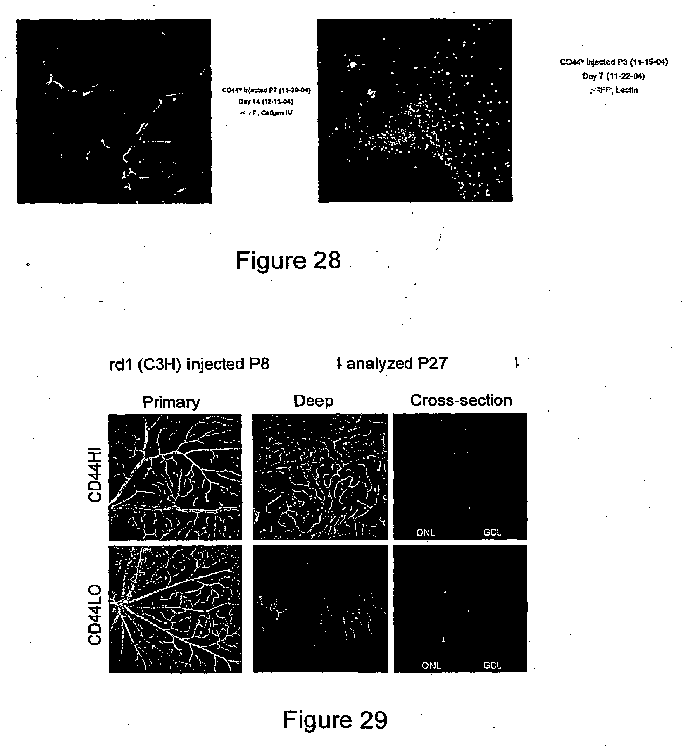

FIG. 28 shows photomicrographic images of a retina from a mouse intravitreally injected with cells from the preferred isolated MLBM cell population (left panel) compared to a retina from a mouse intravitreally injected with CD44^{lo} cells.
FIG. 29 shows photomicrographic images of retinas from eyes injected with cells from the MLBM cell population (CD44^{hi}) and with CD44^{lo} cells.

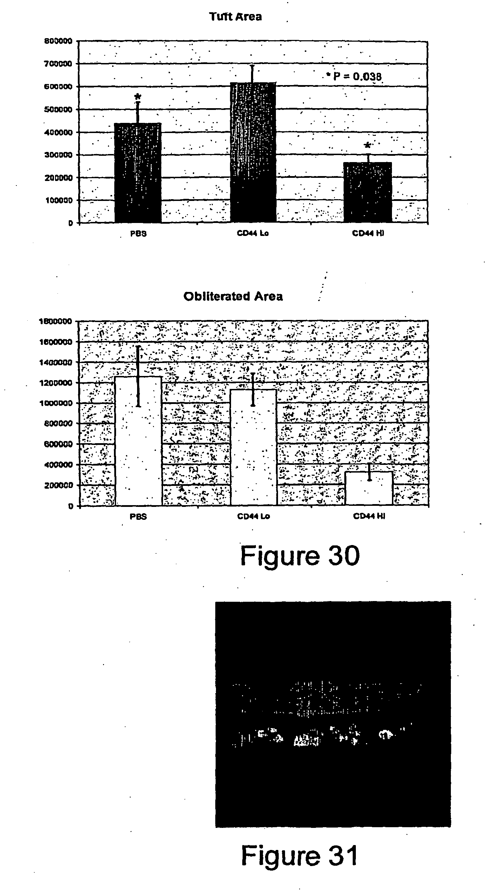

FIG. 30 shows bar graphs demonstrating the beneficial effects of the MLBM cell population for ameliorating pathogenic angiogensis and promoting beneficial physiological revascularization of mouse retinas in the oxygen induced retinopathy model of retinopathy of prematurity. The upper graph compares pre-retinal neovascular tuft area for control retina (first bar), retina treated with CD44^{lo} cells (middle bar) and retinas treated with cells from the MLBM cell population (right bar). The lower graph compares vascular obliteration area for control retina (first bar), retina treated with CD44^{lo} cells (middle bar) and retinas treated with cells from the MLBM cell population (right bar).
FIG. 31 is a photomicrographic image demonstrating that once cells from the MLBM cell population have incorporated into the vasculature of the retina, the cells express vascular endothelial growth factor (VEGF), as indicated by the green staining of the cells in the lower portion of the image.

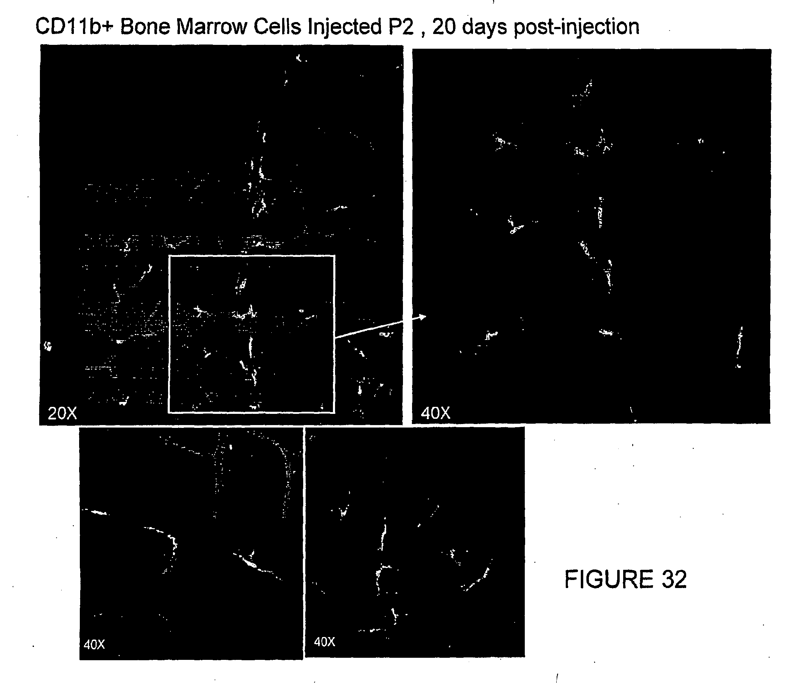

FIG. 32 depicts photomicrographic images demonstrating that cells from the CD11b⁺ MLBM cell population selectively target the vasculature of the retina.

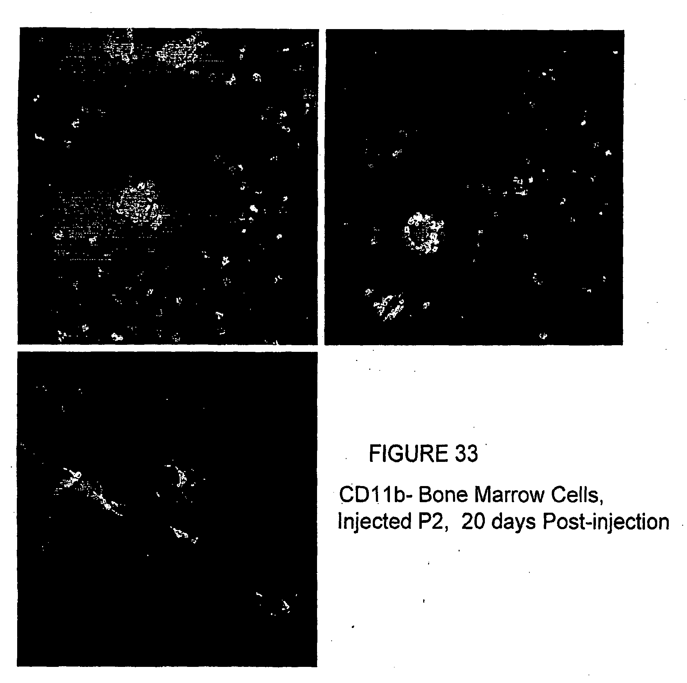

FIG. 33 depicts photomicrographic images demonstrating that CD44⁻ CD11b⁻ bone marrow cells do not selectively target the vasculature of the retina.

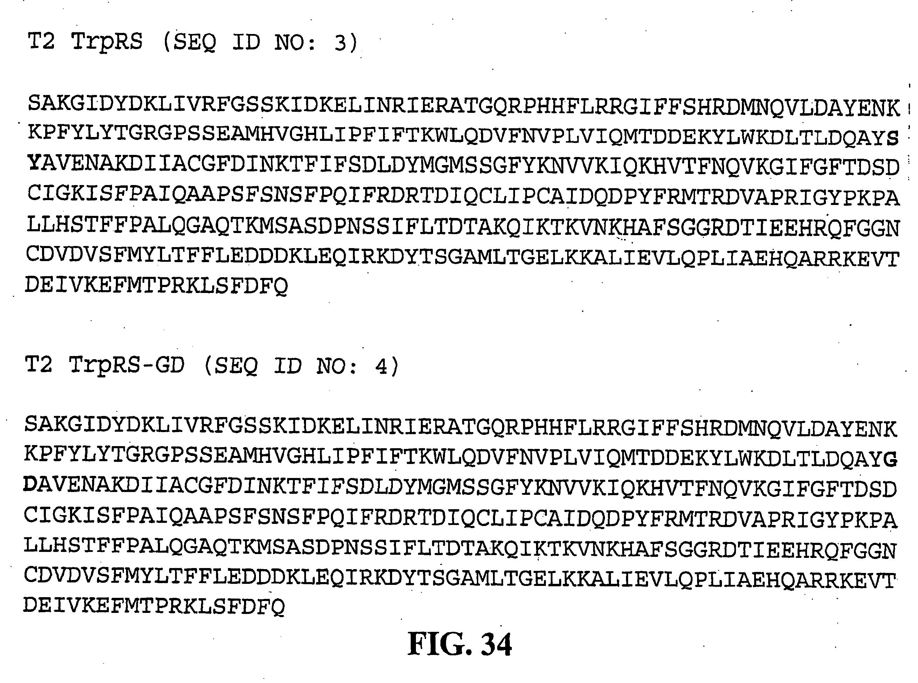

FIG. 34 shows the amino acid residue sequence of the T2 fragment of TrpRS (SEQ ID NO: 3) and of the T2-TrpRS-GD variation thereof (SEQ ID NO: 4).

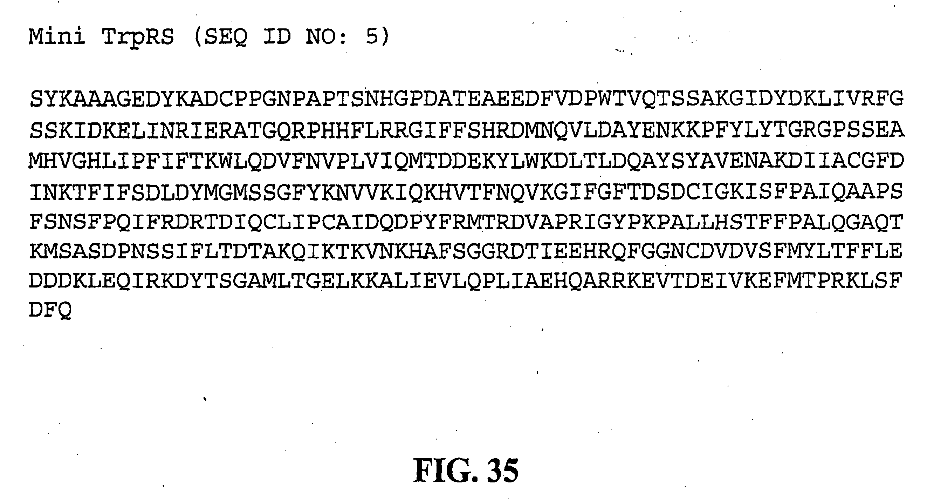

FIG. 35 shows the amino acid residue sequence of mini-TrpRS (SEQ ID NO: 5).

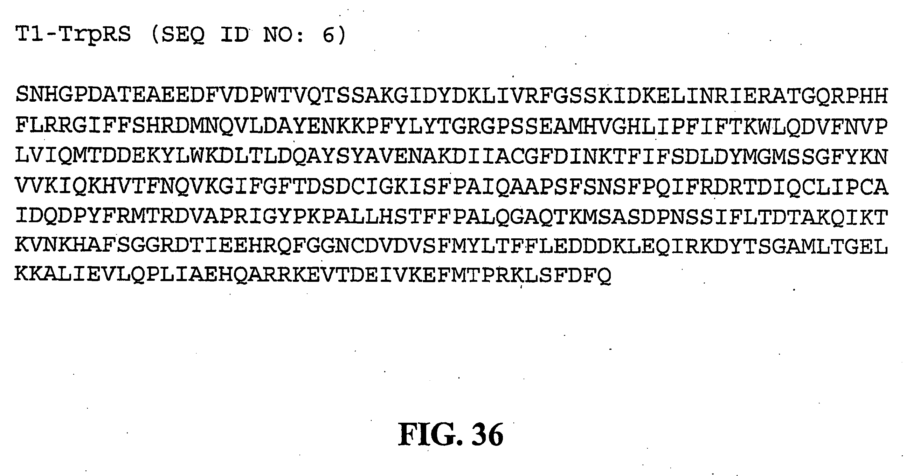

FIG. 36 shows the amino acid residue sequence of T1-TrpRS (SEQ ID NO: 6).

### DETAILED DESCRIPTION OF PREFERRED EMBODIMENTS

Bone marrow includes hematopoietic stem cells that are capable of developing into various blood cell types e.g., B cells, T cells, granulocytes, platelets, and erythrocytes. The lineage surface antigens are a group of cell-surface proteins that are markers of mature blood cell lineages, including CD2, CD3, CD11, CD11a, Mac-1 (CD11b:CD18), CD14, CD16, CD19, CD24, CD33, CD36, CD38, CD45, CD45RA, murine Ly-6G, murine TER-119, CD56, CD64, CD68, CD86 (B7.2), CD66b, human leucocyte antigen DR (HLA-DR), and CD235a (Glycophorin A). Isolated hematopoietic stem cells that do not express significant levels of a "lineage surface antigen" (Lin) on their cell surfaces are referred to herein as "lineage negative" or "Lin⁻" hematopoietic stem cells i.e., Lin⁻ HSC. Human hematopoietic stem cells commonly express other surface antigens such as CD31, CD34, CD117 (c-kit) and/or CD133. Murine hematopoietic stem cells commonly express other surface antigens such as CD34, CD117 (c-kit), Thy-1, and/or Sca-1.

Bone marrow cells include a sub-population of cells that express the CD44 antigen (i.e., the hyaluronic acid receptor) and CD11b (integrin αM). A myeloid-like population of bone marrow cells enriched in CD44 and CD111b expressing cells can be isolated from bone marrow by treating bone marrow cells with an antibody to CD44 antigen (anti-CD44) and/or an antibody to CD11b antigen (anti-CD11b), and then selecting cells that immunoreact with the antibody. The antibody then can be removed from the cells by methods that are well known in the art. The cells can be selected, for example, using by flow cytometry, using antibodies bound to or coated on beads followed by filtration, or other separation methods that are well known in the art. A majority of the selected cells are lineage negative and express both the CD44 antigen and the CD11b antigen, regardless of which antibody is utilized in the isolation.

Stem cells are typically identified by the distribution of antigens on the surface of the cells (for a detailed discussion see Stem Cells: Scientific Progress and Future Directions, a report prepared by the National Institutes of Health, Office of Science Policy, June 2001, Appendix E: Stem Cell Markers, which is incorporated herein by reference to the extent pertinent). Approximately 75% of lineage negative hematopoietic stems cells isolated from bone marrow are also CD44 positive. In a preferred embodiment, a majority of the cells from the MLBM cell population are lineage negative hematopoietic stem cells (i.e., CD44⁺Lin⁻HSC).

As used herein and in the appended claims, the phrase "adult" in reference to bone marrow and bone marrow cells, includes bone marrow isolated postnatally, i.e., from juvenile and adult individuals, as opposed to embryos. Accordingly, the term "adult mammal" refers to both juvenile (postnatal) and fully mature mammals, as opposed to an embryo or prenatal individual.

Lin⁻ HSC populations containing endothelial progenitor cells (EPCs) are particularly useful. The isolated Lin⁻ HSC populations preferably comprise mammalian cells in which at least about 20% of the cells express the surface antigen CD31, which is commonly present on endothelial cells. In other embodiment, at least about 50% of the cells express CD31, more preferably at least about 65%, most preferably at least about 75%. Preferably at least about 50% of the cells of the Lin⁻ HSC populations preferably express the integrin α6 antigen.

In one preferred murine Lin⁻ HSC population, at least about 50% of the cells express CD31 antigen and at least about 50% of the cells express the CD117 (c-kit) antigen. Preferably, at least about 75% of the Lin⁻ HSC cells express the surface antigen CD31, more preferably about 81% of the cells. In another preferred murine embodiment, at least about 65% of the cells express the surface antigen CD117, more preferably about 70% of the cells. A particularly preferred embodiment is a population of Lin⁻ HSCs in which about 50% to about 85% of the cells express the surface antigen CD31 and about 70% to about 75% of the cells express the surface antigen CD117.

In a preferred human Lin⁻ HSC population, the cells are CD133 negative, at least about 50% of the cells express the CD31 surface antigen, and at least about 50% of the cells express the integrin α6 antigen. Yet another preferred embodiment is a human Lin⁻ HSC population in which the cells are CD133 positive, in which at less than about 30% of the cells express the CD31 surface antigen and less than about 30% of the cells express the integrin α6 antigen.

The isolated Lin⁻ HSC populations selectively target astrocytes and incorporate into the retinal neovasculature when intravitreally injected into the eye of the mammalian species, such as a mouse or a human, from which the cells were isolated.

Isolated MLBM cell populations also selectively target astrocytes and incorporate into the retinal neovasculature when intravitreally injected into the eye of the mammalian species, such as a mouse or a human, from which the cells were isolated.

The isolated MLBM cell populations include cells that differentiate to endothelial cells and generate vascular structures within the retina. In particular, MLBM cell populations are useful for the treatment of retinal neovascular and retinal vascular degenerative diseases, and for repair of retinal vascular injury. MLBM cell populations also promote neuronal rescue in the retina and promote upregulation of anti-apoptotic genes. The MLBM cell populations are particularly useful for treating retinal defects in the eyes of neonatal mammals, such as mammals suffering from oxygen induced retinopathy or retinopathy of prematurity.

The number of cells injected into the eye is sufficient for arresting the disease state of the eye. For example, the amount of injected cells can be effective for repairing retinal damage of the eye, stabilizing retinal neovasculature, maturing retinal neovasculature, and preventing or repairing vascular leakage and vascular hemorrhage.

Anti-angiogenic (i.e., angiostatic) fragments of TrpRS are, e.g., the T1 and T2 fragments of TrpRS, which are described in detail in co-owned, co-pending U.S. patent application Serial No. 10/080,839. The transfected cells used according to the present invention are useful for treatment of retinal diseases involving abnormal vascular development, such as diabetic retinopathy, and like diseases. Preferably, the cell populations are those of human cells.

The transfected cells may include a gene operably encoding a neurotrophic agent such as nerve growth factor, neurotrophin-3, neurotrophin-4, neurotrophin-5, ciliary neurotrophic factor, retinal pigmented epithelium-derived neurotrophic factor, insulin-like growth factor, glial cell line-derived neurotrophic factor, brain-derived neurotrophic factor, and the like. Such neurotrophic cells are useful for promoting neuronal rescue in retinal neuronal degenerative diseases such as glaucoma and retinitis pigmentosa, in treatment of injuries to the retinal nerves, and the like. Implants of ciliary neurotrophic factor have been reported as useful for the treatment of retinitis pigmentosa (see Kirby et al. 2001, Mol Ther. 3(2):241-8; Farrar et al. 2002, EMBO Journal 21:857-864). Brain-derived neurotrophic factor reportedly modulates growth associated genes in injured retinal ganglia (see Fournier, et al., 1997, J. Neurosci. Res. 47:561-572). Glial cell line derived neurotrophic factor reportedly delays photoreceptor degeneration in retinitis pigmentosa (see McGee et al. 2001, Mol Ther. 4(6):622-9).

Preferably, at least about 1 x 10⁵ cells are administered by intravitreal injection to a mammalian eye suffering from a retinal degenerative disease. The amount of cells to be injected may depend upon the severity of the retinal degeneration, the age of the mammal and other factors that will be readily apparent to one of ordinary skill in the art of treating retinal diseases. The cells may be administered in a single dose or by multiple dose administration over a period of time, as determined by the clinician in charge of the treatment.

Results from the OIR model of ROP indicate that a particular advantage is a vasculotrophic and neurotrophic rescue effect observed in eyes intravitreally treated with cells from the lineage negative hematopoietic stem cell populations. Retinal neurons and photoreceptors, particularly cones, are preserved and some measure of visual function can be maintained in eyes treated with cells from the lineage negative hematopoietic stem cell populations. Treatment with angiogenesis inhibitors did not block the therapeutic effects of the lineage negative hematopoietic stem cell populations. Macrophage-like cells were observed in conjunction with the rescued blood vessels, suggesting a possible link between immune cells and the lineage negative bone marrow cells in the vasculotrophic activity of the lineage negative cells. Injection of antibodies to CD 18 between P7 and P 17, to reduce macrophage extravasation from the blood vessels did not block the rescue effects of the lineage negative hematopoietic stem cells, however.

Cells may be injected prior to the onset of the disease or at early stages thereof to protect the retina from damage that would otherwise ensue if the eye were left untreated. Such prophylactic treatment is particularly desirable in cases where the mammal to be treated is known to be at risk of developing retinopathy of prematurity, oxygen induced retinopathy, or other retinopathic diseases. Prophylactic treatments are particularly effective, since the presence of the lineage negative cell populations in the eye actually lessens the severity of vascular damage to the retina, and may arrest the disease before damage is done, as opposed to merely promoting recovery from damage. For example, in the OIR model, mice injected with Lin⁻ HSC between P3 and P7, prior to hyperoxic exposure, suffer less retinal damage and recover fast than mice injected after hyperoxic exposure. Furthermore, mice that were treated during hyperoxic exposure also demonstrated accelerated recovery.

### Murine Retinal Vascular Development.

*A Model for Ocular Angiogenesis.* The mouse eye provides a recognized model for the study of mammalian retinal vascular development, such as human retinal vascular development. During development of the murine retinal vasculature, ischemia-driven retinal blood vessels develop in close association with astrocytes. These glial elements migrate onto the third trimester human fetus, or the neonatal rodent, retina from the optic disc along the ganglion cell layer and spread radially. As the murine retinal vasculature develops, endothelial cells utilize this already established astrocytic template to determine the retinal vascular pattern (*See* FIG. 1 (a and b)). FIG. 1 (a and b) depicts schematic diagrams of developing mouse retina. Panel (a) depicts development of the primary plexus (dark lines at upper left of the diagram) superimposed over the astrocyte template (light lines) whereas, (b) depicts the second phase of retinal vessel formation. In FIG. 1, GCL stands for ganglion cell layer; IPL stands for inner plexus layer; INL stands for inner nuclear layer; OPL stands for outer plexus layer; ONL stands for outer nuclear layer; RPE stands for retinal pigment epithelium; ON stands for optic nerve; and P stands for periphery.

At birth, retinal vasculature is virtually absent. By postnatal day 14 (P14) the retina has developed complex primary (superficial) and secondary (deep) layers of retinal vessels coincident with the onset of vision. Initially, spoke-like peripapillary vessels grow radially over the pre-existing astrocytic network towards the periphery, becoming progressively interconnected by capillary plexus formation. These vessels grow as a monolayer within the nerve fiber through P10 (FIG. 1 (a)). Between P7-P8 collateral branches begin to sprout from this primary plexus and penetrate into the retina to the outer plexiform layer where they form the secondary, or deep, retinal plexus. By P21, the entire network undergoes extensive remodeling and a tertiary, or intermediate, plexus forms at the inner surface of inner nuclear layer (FIG. 1 (b)).

The neonatal mouse retinal angiogenesis model is useful for studying the role of HSC during ocular angiogenesis for several reasons. In this physiologically relevant model, a large astrocytic template exists prior to the appearance of endogenous blood vessels, permitting an evaluation of the role for cell-cell targeting during a neovascular process. In addition, this consistent and reproducible neonatal retinal vascular process is known to be hypoxia-driven, in this respect having similarities to many retinal diseases in which ischemia is known to play a role.

### Enrichment of Endothelial Progenitor Cells (EPC) From Bone Marrow.

Although cell surface marker expression has been extensively evaluated on the EPC population found in preparations of HSC, markers that uniquely identify EPC are still poorly defined. To enrich for EPC, hematopoietic lineage marker positive cells (Lin⁺), i.e., B lymphocytes (CD45), T lymphocytes (CD3), granulocytes (Ly-6G), monocytes (CD11), and erythrocytes (TER-119), were depleted from bone marrow mononuclear cells of mice. Sca-1 antigen was used to further enrich for EPC. A comparison of results obtained after intravitreal injection of identical numbers of either Lin⁻ Sca-1⁺ cells or Lin⁻ cells, no difference was detected between the two groups. In fact, when only Lin⁻Sca-1⁻ cells were injected, far greater incorporation into developing blood vessels was observed.

Lin⁻HSC populations are enriched with EPCs, based on functional assays. Furthermore, Lin⁺HSC populations functionally behave quite differently from the Lin⁻ HSC populations. Epitopes commonly used to identify EPC for each fraction (based on previously reported *in vitro* characterization studies) were also evaluated. While none of these markers were exclusively associated with the Lin⁻ fraction, all were increased about 70 to about 1800% in the Lin⁻ HSC, compared to the Lin⁺HSC fraction (FIG. 1 (c)). FIG. 1, Panel (c) illustrates flow cytometric characterization of bone marrow-derived Lin⁺ HSC and Lin⁻ HSC separated cells. The top row of Panel (c) shows a hematopoietic stem cell dot plot distribution of non-antibody labeled cells. R1 defines the quantifiable-gated area of positive PE-staining; R2 indicates GFP-positive. Dot plots of Lin⁻ HSC are shown in the middle row and dot plots of Lin⁺ HSC are shown in the bottom row. The C57B/6 cells were labeled with the PE-conjugated antibodies for Sca-1, c-kit, Flk-1/KDR, CD31. Tie-2 data was obtained from Tie-2-GFP mice. The percentages in the corners of the dot plots indicate the percent of positive-labeled cells out of total Lin⁻ or Lin⁺ HSC population. Interestingly, accepted EPC markers like Flk-1/KDR, Tie-2, and Sca-1 were poorly expressed and, thus, not used for further fractionation.

Lin⁻ HSC can be isolated by (a) extracting bone marrow from an adult mammal; (b) separating a plurality of monocytes from the bone marrow; (c) labeling the monocytes with biotin-conjugated lineage panel antibodies to one or more lineage surface antigens, preferably lineage surface antigens selected from the group consisting of CD2, CD3, CD4, CD11, CD11a, Mac-1, CD 14, CD16, CD19, CD24, CD33, CD36, CD38, CD45, Ly-6G (murine), TER-119 (murine), CD45RA, CD56, CD64, CD68, CD86 (B7.2), CD66b, human leucocyte antigen DR (HLA-DR), and CD235a (Glycophorin A); (d) removing monocytes that are positive for said one or more lineage surface antigens from the plurality of monocytes; and (e) recovering a population of lineage negative hematopoietic stem cells therefrom.

When the Lin⁻ HSC are isolated from adult human bone marrow, preferably the monocytes are labeled with biotin-conjugated lineage panel antibodies to lineage surface antigens CD2, CD3, CD4, CD11a, Mac-1, CD14, CD16, CD19, CD33, CD38, CD45RA, CD64, CD68, CD86 (B7.2), and CD235a. When the Lin⁻ HSC are isolated from adult murine bone marrow, preferably the monocytes are labeled with biotin-conjugated lineage panel antibodies to lineage surface antigens CD3, CD11, CD45, Ly-6G, and TER-119.

### Intravitreally Injected HSC Lin⁻ Cells Contain EPC That Target Astrocytes and Incorporate into Developing Retinal Vasculature.

To determine whether intravitreally injected Lin⁻ HSC can target specific cell types of the retina, utilize the astrocytic template and participate in retinal angiogenesis, approximately 10⁵ cells from a Lin⁻ HSC composition or Lin⁺ HSC cells (control, about 10⁵ cells) isolated from the bone marrow of adult (GFP or LacZ transgenic) mice were injected into postnatal day 2 (P2) mouse eyes. Four days after injection (P6), many cells from the Lin⁻ HSC composition, derived from GFP or LacZ transgenic mice were adherent to the retina and had the characteristic elongated appearance of endothelial cells (FIG. 2 (a)). FIG. 2 illustrates engraftment of Lin⁻ cells into developing mouse retina. As shown in FIG. 2, Panel (a), the four days post-injection (P6) intravitreally injected eGFP+ Lin⁻ HSC attach and differentiate on the retina.

In many regions of the retinas, the GFP-expressing cells were arranged in a pattern conforming to underlying astrocytes and resembled blood vessels. These fluorescent cells were observed ahead of the endogenous, developing vascular network (FIG. 2 (b)). Conversely, only a small number of Lin⁺HSC (FIG. 2 (c)), or adult mouse mesenteric endothelial cells (FIG. 2 (d)) attached to the retinal surface. In order to determine whether cells from an injected Lin⁻ HSC population could also attach to retinas with already established vessels, a Lin⁻ HSC composition was injected into adult eyes. Interestingly, no cells were observed to attach to the retina or incorporate into established, normal retinal blood vessels (FIG. 2 (e)). This indicates that the Lin⁻ HSC compositions do not disrupt a normally developed vasculature and will not initiate abnormal vascularization in normally developed retinas.

In order to determine the relationship between injected Lin⁻ HSC compositions and retinal astrocytes, a transgenic mouse was used, which expressed glial fibrillary acidic protein (GFAP, a marker of astrocytes) and promoter-driven green fluorescent protein (GFP). Examination of retinas of these GFAP-GFP transgenic mice injected with Lin⁻ HSC from eGFP transgenic mice demonstrated co-localization of the injected eGFP EPC and existing astrocytes (FIG. 2 (f-h), arrows). Processes of eGFP+Lin⁻ HSC were observed to conform to the underlying astrocytic network (arrows, FIG. 2 (g)). Examination of these eyes demonstrated that the injected, labeled cells only attached to astrocytes; in P6 mouse retinas, where the retinal periphery does not yet have endogenous vessels, injected cells were observed adherent to astrocytes in these not yet vascularized areas. Surprisingly, injected, labeled cells were observed in the deeper layers of the retina at the precise location where normal retinal vessels will subsequently develop (FIG. 2 (i), arrows).

To determine whether injected Lin⁻ HSC are stably incorporated into the developing retinal vasculature, retinal vessels at several later time points were examined. As early as P9 (seven days after injection), Lin⁻ HSC incorporated into CD31⁺structures (FIG. 2 (j)). By P16 (14 days after injection), the cells were already extensively incorporated into retinal vascular-like structures (FIG. 2 (k)). When rhodamine-dextran was injected intravascularly (to identify functional retinal blood vessels) prior to sacrificing the animals, the majority of Lin⁻ HSC were aligned with patent vessels (FIG. 2 (1)). Two patterns of labeled cell distribution were observed: (1) in one pattern, cells were interspersed along vessels in between unlabeled endothelial cells; and (2) the other pattern showed that vessels were composed entirely of labeled cells. Injected cells were also incorporated into vessels of the deep vascular plexus (FIG. 2 (m)). While sporadic incorporation of Lin⁻ HSC-derived EPC into neovasculature has been previously reported, this is the first report of vascular networks being entirely composed of these cells. This demonstrates that cells from a population of bone marrow-derived Lin⁻ HSC, injected intravitreally, can efficiently incorporate into any layer of the forming retinal vascular plexus.

Histological examination of non-retinal tissues (e.g., brain, liver, heart, lung, bone marrow) did not demonstrate the presence of any GFP positive cells when examined up to 5 or 10 days after intravitreal injection. This indicates that a sub-population of cells within the Lin⁻ HSC fraction selectively target to retinal astrocytes and stably incorporate into developing retinal vasculature. Since these cells have many characteristics of endothelial cells (association with retinal astrocytes, elongate morphology, stable incorporation into patent vessels and not present in extravascular locations), these cells represent EPC present in the Lin⁻ HSC population. The targeted astrocytes are of the same type observed in many of the hypoxic retinopathies. It is well known that glial cells are a prominent component of neovascular fronds of tufts observed in DR and other forms of retinal injury. Under conditions of reactive gliosis and ischemia-induced neovascularization, activated astrocytes proliferate, produce cytokines, and up-regulate GFAP, similar to that observed during neonatal retinal vascular template formation in many mammalian species including humans.

Lin⁻ HSC populations will target activated astrocytes in adult mouse eyes as they do in neonatal eyes, Lin⁻ HSC cells were injected into adult eyes with retinas injured by photo-coagulation (FIG. 3 (a)) or needle tip (FIG. 3 (b)). In both models, a population of cells with prominent GFAP staining was observed only around the injury site (FIG. 3 (a and b)). Cells from injected Lin⁻ HSC compositions localized to the injury site and remained specifically associated with GFAP-positive astrocytes (FIG. 3 (a and b)). At these sites, Lin⁻ HSC cells were also observed to migrate into the deeper layer of retina at a level similar to that observed during neonatal formation of the deep retinal vasculature. Uninjured portions of retina contained no Lin⁻ HSC cells, identical to that observed when Lin⁻ HSC were injected into normal, uninjured adult retinas (FIG. 2 (e)). These data indicate that Lin⁻ HSC compositions can selectively target activated glial cells in injured adult retinas with gliosis as well as neonatal retinas undergoing vascularization.

### Intravitreally Injected Lin⁻ HSC Can Rescue and Stabilize Degenerating Vasculature.

Since intravitreally injected Lin⁻ HSC compositions target astrocytes and incorporate into the normal retinal vasculature, these cells also stabilize degenerating vasculature in ischemic or degenerative retinal diseases associated with gliosis and vascular degeneration. The *rd*/*rd* mouse is a model for retinal degeneration that exhibits profound degeneration of photoreceptor and retinal vascular layers by one month after birth. The retinal vasculature in these mice develops normally until P 16 at which time the deeper vascular plexus regresses; in most mice the deep and intermediate plexuses have nearly completely degenerated by P30.

To determine whether HSC can rescue the regressing vessels, Lin⁺ or Lin⁻ HSC (from Balb/c mice) were injected into *rd*/*rd* mice intravitreally at P6. By P33, after injection with Lin⁺ cells, vessels of the deepest retinal layer were nearly completely absent (FIG. 4 (a and b)). In contrast, most Lin⁻ HSC-injected retinas by P33 had a nearly normal retinal vasculature with three parallel, well-formed vascular layers (FIG. 4 (a and d)). Quantification of this effect demonstrated that the average length of vessels in the deep vascular plexus of Lin⁻ injected *rd*/*rd* eyes was nearly three times greater than untreated or Lin⁺ cell-treated eyes (FIG. 4 (e)). Surprisingly, injection of a Lin⁻ HSC composition derived from *rd*/*rd* adult mouse (FVB/N) bone marrow also rescued degenerating *rd*/*rd* neonatal mouse retinal vasculature (FIG. 4 (f)). Degeneration of the vasculature in *rd*/*rd* mouse eyes in observed as early as 2-3 weeks post-natally. Injection of Lin⁻ HSC as late as P15 also resulted in partial stabilization of the degenerating vasculature in the *rd*/*rd* mice for at least one month (FIG. 4 (g and h)).

A Lin⁻ HSC composition injected into younger (e.g., P2) *rd*/*rd* mice also incorporated into the developing superficial vasculature. By P11, these cells were observed to migrate to the level of the deep vascular plexus and form a pattern identical to that observed in the wild type outer retinal vascular layer (FIG. 5 (a)). In order to more clearly describe the manner in which cells from injected Lin⁻ HSC compositions incorporate into, and stabilize, degenerating retinal vasculature in the *rd*/*rd mice,* a Lin⁻ HSC composition derived from Balb/c mice was injected into Tie-2-GFP FVB mouse eyes. The FVB mice have the *rd*/*rd* genotype and because they express the fusion protein Tie-2-GFP, all endogenous blood vessels are fluorescent.

When non-labeled cells from a Lin⁻ HSC composition are injected into neonatal Tie-2-GFP FVB eyes and are subsequently incorporated into the developing vasculature, there should be non-labeled gaps in the endogenous, Tie-2-GFP labeled vessels that correspond to the incorporated, non-labeled Lin⁻ HSC that was injected. Subsequent staining with another vascular marker (e.g., CD-31) then delineates the entire vessel, permitting determination as to whether non-endogenous endothelial cells are part of the vasculature. Two months after injection, CD31-positive, Tie-2-GFP negative, vessels were observed in the retinas of eyes injected with the Lin⁻ HSC composition (FIG. 5 (b)). Interestingly, the majority of rescued vessels contained Tie-2-GFP positive cells (FIG. 5 (c)). The distribution of pericytes, as determined by staining for smooth muscle actin, was not changed by Lin⁻ HSC injection, regardless of whether there was vascular rescue (FIG. 5 (d)). These data clearly demonstrate that intravitreally injected Lin⁻ HSC cells migrate into the retina, participate in the formation of normal retinal blood vessels, and stabilize endogenous degenerating vasculature in a genetically defective mouse.

### Inhibition of Retinal Angiogenesis by Transfected Cells from Lin⁻HSC.

The majority of retinal vascular diseases involve abnormal vascular proliferation rather than degeneration. Transgenic cells targeted to astrocytes can be used to deliver an anti-angiogenic protein and inhibit angiogenesis. Cells from Lin⁻ HSC compositions were transfected with T2-tryptophanyl-tRNA synthetase (T2-TrpRS). T2-TrpRS is a 43 kD fragment of TrpRS that potently inhibits retinal angiogenesis (FIG. 6 (a) and FIG. 34). On P12, retinas of eyes injected with a control plasmid-transfected Lin⁻ HSC composition (no T2-TrpRS gene) on P2 had normal primary (FIG. 6 (c)) and secondary (FIG. 6 (d)) retinal vascular plexuses. When the T2-TrpRS transfected Lin⁻ HSC composition was injected into P2 eyes and evaluated 10 days later, the primary network had significant abnormalities (FIG. 6 (e)) and formation of the deep retinal vasculature was nearly completely inhibited (FIG. 6 (f)). The few vessels observed in these eyes were markedly attenuated with large gaps between vessels. The extent of inhibition by T2-TrpRS-secreting Lin⁻ HSCs is detailed in Table 1.

T2-TrpRS is produced and secreted by cells in the Lin⁻ HSC composition *in vitro* and after injection of these transfected cells into the vitreous, a 30 kD fragment of T2-TrpRS in the retina (FIG. 6 (b)) was observed. This 30 kD fragment was specifically observed only in retinas injected with transfected Lin⁻ HSC and this decrease in apparent molecular weight compared to the recombinant or *in vitro*-synthesized protein may be due to processing or degradation of the T2-TrpRS *in vivo.* These data indicate that Lin⁻ HSC compositions can be used to deliver functionally active genes, such as genes expressing angiostatic molecules, to the retinal vasculature by targeting to activated astrocytes. While it is possible that the observed angiostatic effect is due to cell-mediated activity this is very unlikely since eyes treated with identical, but non-T2-transfected Lin⁻ HSC compositions had normal retinal vasculature.

**Table 1. Vascular Inhibition by T2-TrpRS-secreting Lin⁻HSCs**

| | **Primary Plexus** | | **Deep Plexus** | | |
|---|---|---|---|---|---|
| | **Inhibited** | **Normal** | **Complete** | **Partial** | **Normal** |
| T2-TrpRS | 60% | 40% | 33.3% | 60% | 6.7% |
| (15 eyes) | (9 eyes) | (6 eyes) | (5 eyes) | (9 eyes) | (1 eye) |
| Control | 0% | 100% | 0% | 38.5% | 61.5% |
| (13 eyes) | (0 eyes) | (13 eyes) | (0 eyes) | (5 eyes) | (8 eyes) |

Intravitreally injected Lin⁻ HSC populations localize to retinal astrocytes, incorporate into vessels, and can be useful in treating many retinal diseases. While most cells from injected HSC compositions adhere to the astrocytic template, small numbers migrate deep into the retina, homing to regions where the deep vascular network will subsequently develop. Even though no GFAP-positive astrocytes were observed in this area prior to 42 days postnatally, this does not rule out the possibility that GFAP-negative glial cells are already present to provide a signal for Lin⁻ HSC localization. Previous studies have shown that many diseases are associated with reactive gliosis. In DR, in particular, glial cells and their extracellular matrix are associated with pathological angiogenesis.

Since cells from injected Lin⁻ HSC compositions specifically attached to GFAP-expressing glial cells, regardless of the type of injury, Lin⁻ HSC compositions can be used to target pre-angiogenic lesions in the retina. For example, in the ischemic retinopathies, such as diabetes, neovascularization is a response to hypoxia. By targeting Lin⁻ HSC compositions to sites of pathological neovascularization, developing neovasculature can be stabilized preventing abnormalities of neovasculature such as hemorrhage or edema (the causes of vision loss associated with DR) and can potentially alleviate the hypoxia that originally stimulated the neovascularization. Abnormal blood vessels can be restored to normal condition. Furthermore, angiostatic proteins, such as T2-TrpRS (SEQ ID NO: 3 in FIG. 34), T2-TrpRS-GD (SEQ ID NO: 4 in FIG. 34), mini-TrpRS (SEQ ID NO: 5 in FIG. 35), and T1-TrpRS (SEQ ID NO: 6 in FIG. 36) can be delivered to sites of pathological angiogenesis by using transfected Lin⁻ HSC compositions and laser-induced activation of astrocytes. Preferred angiostatic fragments of TrpRS include T2-TrpRS and T2-TrpRS-GD. Since laser photocoagulation is commonly used in clinical ophthalmology, this approach has application for many retinal diseases. While such cell-based approaches have been explored in cancer therapy, their use for eye diseases is more advantageous since intraocular injection makes it possible to deliver large numbers of cells directly to the site of disease.

### Neurotrophic and Vasculotrophic Rescue by Lin⁻HSC.

MACS was used to separate Lin⁻ HSC from bone marrow of enhanced green fluorescent protein (eGFP), C3H (*rd*/*rd*), FVB (*rd*/*rd*) mice as described above. Lin⁻ HSC containing EPC from these mice were injected intravitreally into P6 C3H or FVB mouse eyes. The retinas were collected at various time points (1 month, 2 months, and 6 months) after injection. The vasculature was analyzed by scanning laser confocal microscope after staining with antibodies to CD31 and retinal histology after nuclear staining with DAPI. Microarray gene expression analysis of mRNA from retinas at varying time points was also used to identify genes potentially involved in the effect.

Eyes of *rd*/*rd* mice had profound degeneration of both neurosensory retina and retinal vasculature by P21. Eyes of *rd*/*rd* mice treated with Lin⁻ HSC on P6 maintained a normal retinal vasculature for as long as 6 months; both deep and intermediate layers were significantly improved when compared to the controls at all time points (1M, 2M, and 6M) (see FIG. 12). In addition, we observed that retinas treated with Lin⁻HSC were also thicker (1M; 1.2-fold, 2M; 1.3-fold, 6M; 1.4-fold) and had greater numbers of cells in the outer nuclear layer (1M; 2.2-fold, 2M; 3.7-fold, 6M; 5.7-fold) relative to eyes treated with Lin⁺ HSC as a control. Large scale genomic analysis of "rescued" (e.g., Lin⁻ HSC) compared to control (untreated or non-Lin⁻ treated) *rd*/*rd* retinas demonstrated a significant upregulation of genes encoding sHSPs (small heat shock proteins) and specific growth factors that correlated with vascular and neural rescue, including genes encoding the proteins listed in FIG. 20, panels A and B.

The bone marrow derived Lin⁻HSC populations significantly and reproducibly induced maintenance of a normal vasculature and dramatically increased photoreceptor and other neuronal cell layers in the *rd*/*rd* mouse. This neurotrophic rescue effect correlated with significant upregulation of small heat shock proteins and growth factors and provides insights into therapeutic approaches to currently untreatable retinal degenerative disorders.

### Rd1/rd1 Mouse Retinas Exhibit Profound Vascular and Neuronal Degeneration.

Normal postnatal retinal vascular and neuronal development in mice has been well described and is analogous to changes observed in the third trimester human fetus (Dorrell et al., 2002, Invest. Ophthalmol. Vis. Sci. 43:3500-3510). Mice homozygous for the *rd1* gene share many characteristics of human retinal degeneration (Frasson et al., 1999, Nat. Med. 5:1183-1187) and exhibit rapid photoreceptor (PR) loss accompanied by severe vascular atrophy as the result of a mutation in the gene encoding PR cGMP phosphodiesterase (Bowes et al. 1990, Nature 347:677-680). To examine the vasculature during retinal development and its subsequent degeneration, antibodies against collagen IV (CIV), an extracellular matrix (ECM) protein of mature vasculature, and CD31 (PECAM-1), a marker for endothelial cells, were used (FIG. 15). Retinas of *rd1lrd1* (C3H/HeJ) developed normally until approximately postnatal day (P) 8 when degeneration of the photoreceptor-containing outer nuclear layer (ONL) began. The ONL rapidly degenerated and cells died by apoptosis such that only a single layer of nuclei remained by P20. Double staining of the whole-mounted retinas with antibodies to both CIV and CD31 revealed details of the vascular degeneration in *rd1*/*rd1* mice similar to that described by others (Blanks et al., 1986, J Comp. Neurol. 254:543-553). The primary and deep retinal vascular layers appeared to develop normally though P12 after which there is a rapid loss of endothelial cells as evidenced by the absence of CD31 staining. CD31 positive endothelial cells were present in a normal distribution through P12 but rapidly disappeared after that. Interestingly, CIV positive staining remained present throughout the time points examined, suggesting that the vessels and associated ECM formed normally, but only the matrix remained after P 13 by which time no CD31 positive cells were observed. (FIG. 15, middle panels). The intermediate vascular plexus also degenerates after P21, but the progression is slower than that observed in the deep plexus (FIG. 15, upper panel). Retinal vascular and neural cell layers of a normal mouse are shown for comparison to the *rd1*/*rd1* mouse (right panels, FIG. 15).

### Neuroprotective Effect of Bone Marrow-Derived Lin⁻ HSCs in rd1/rd1 Mice.

Intravitreally injected Lin⁻ HSCs incorporate into endogenous retinal vasculature in all three vascular plexuses and prevent the vessels from degenerating. Interestingly, the injected cells are virtually never observed in the outer nuclear layer. These cells either incorporate into the forming retinal vessels or are observed in close proximity to these vessels. Murine Lin⁻ HSCs (from C3H/HeJ) were intravitreally injected into C3H/HeJ (*rd1*/*rd1*) mouse eyes at P6, just prior to the onset of degeneration. By P30, control cell (CD31⁻)-injected eyes exhibited the typical *rd1*/*rd1* phenotype, i.e., nearly complete degeneration of the deep vascular plexus and ONL was observed in every retina examined. Eyes injected with Lin⁻ HSCs maintained normal-appearing intermediate and deep vascular plexuses. Surprisingly, significantly more cells were observed in the internuclear layer (INL) and ONL of Lin⁻ HSC-injected eyes than in control cell-injected eyes (FIG. 16 (A)). This rescue effect of Lin⁻ HSCs could be observed at 2 months (FIG. 16 (B)) and for as long as 6 months after injection (FIG. 16 (C)). Differences in the vasculature of the intermediate and deep plexuses of Lin⁻ HSC-injected eyes, as well as the neuronal cell-containing INL and ONL, were significant at all time points measured when rescued and non-rescued eyes were compared (FIG. 16 (B and C)). This effect was quantified by measuring the total length of the vasculature (FIG. 16 (D)) and counting the number of DAPI-positive cell nuclei observed in the ONL (FIG. 16 (E)). Simple linear-regression analysis was applied to the data at all time points.

A statistically significant correlation was observed between vascular rescue and neuronal (e.g., ONL thickness) rescue at P30 (*p* < 0.024) and P60 *(p* < 0.034) in the Lin⁻ HSC-injected eyes (FIG. 16 (F)). The correlation remained high, although not statistically significant (*p*< 0.14) at P 180 when comparing Lin⁻ HSC-injected retinas to control cell-injected retinas (FIG. 16 (F)). In contrast, control cell-injected retinas showed no significant correlation between the preservation of vasculature and ONL at any time point (FIG. 16 (F)). These data demonstrate that intravitreal injection of Lin⁻ HSCs results in concomitant retinal vascular and neuronal rescue in retinas of *rd1*/*rd1* mice. Injected cells were not observed in the ONL or any place other than within, or in close proximity to, retinal blood vessels.

### Functional Rescue of Lin⁻ HSC-injected rd/rd Retinas.

Electroretinograms (ERGs) were performed on mice 2 months after injection of control cells or murine Lin⁻ HSCs (FIG. 17). Immunohistochemical and microscopic analysis was done with each eye following ERG recordings to confirm that vascular and neuronal rescue had occurred. Representative ERG recordings from treated, rescued and control, non-rescued eyes show that in the rescued eyes, the digitally subtracted signal (treated minus untreated eyes) produced a clearly detectable signal with an amplitude on the order of 8-10 microvolts (FIG. 17). Clearly, the signals from both eyes are severely abnormal. However, consistent and detectable ERGs were recordable from the Lin⁻ HSC-treated eyes. In all cases the ERG from the control eye was non-detectable. While the amplitudes of the signals in rescued eyes were considerably lower than normal, the signals were consistently observed whenever there was histological rescue and were on the order of magnitude of those reported by other, gene based, rescue studies. Overall these results are demonstrate of some degree of functional rescue in the eyes treated with the Lin⁻ HSCs.

### Rescued rd/rd retinal cell types are predominantly cones.

Rescued and non-rescued retinas were analyzed immunohistochemically with antibodies specific for rod or cone opsin. The same eyes used for the ERG recordings presented in FIG. 17 were analyzed for rod or cone opsin. In wild type mouse retinas, less than about 5% of photoreceptors present are cones (Soucy et al. 1998, Neuron 21: 481-493) and the immunohistochemical staining patterns observed with red/green cone opsin as shown in FIG. 25 (A) or rod rhodopsin as shown in FIG. 25 (B), were consistent with this percentage of cone cells. When wild type retinas were stained with pre-immune IgG, no staining was observed anywhere in the neurosensory retinas other than autoflouresence of the blood vessels (FIG. 25 (C)). Two months after birth, retinas of non-injected rd/rd mice had an essentially atrophic outer nuclear layer that does not exhibit any staining with antibodies to red green cone opsin (FIG. 25 (D)) or rhodopsin (FIG. 25 (G)). Eyes injected with control, CD31⁻ HSC also did not stain positively for the presence of either cone (FIG. 25 (E))) or rod (FIG. 25 (H)) opsin. In contrast, contralateral eyes injected with Lin⁻HSC had about 3 to about 8 rows of nuclei in a preserved outer nuclear layer; most of these cells were positive for cone opsin (FIG. 25 (F)) with approximately 1-3% positive for rod opsin (FIG. 25 (I)). Remarkably, this is nearly the reverse of what is ordinarily observed in the normal mouse retina, which is rod-dominated. These data demonstrate that the injection of Lin⁻ HSC preserves cones for extended periods of time during which they would ordinarily degenerate.

### Human bone marrow (hBM)-derived Lin⁻ HSCs also Rescue Degenerating Retinas.

Lin⁻ HSCs isolated from human bone marrow behave similarly to murine Lin⁻ HSCs. Bone marrow was collected from human donors and the Lin⁺ HSCs were depleted, producing a population of human Lin⁻ HSCs (hLin⁻ HSCs). These cells were labeled *ex-vivo* with fluorescent dye and injected into C3SnSmn.CB17-*Prkdc* SCID mouse eyes. The injected hLin⁻ HSCs migrated to, and targeted, sites of retinal angiogenesis in a fashion identical to that observed when murine Lin⁻ HSCs were injected (FIG. 18 (A)). In addition to the vascular targeting, the human Lin⁻ HSCs also provided a robust rescue effect on both the vascular and neuronal cell layers of the *rd1*/*rd1* mice (FIG. 18 (B and C)). This observation confirms the presence of cells in human bone marrow that target retinal vasculature and can prevent retinal degeneration.

### Lin⁻ HSCs have Vasculo- and Neurotrophic Effects in the rd10/rd10 Mouse.

While *the rd1lrd1* mouse is the most widely used and best characterized model for retinal degeneration (Chang et al. 2002, Vision Res. 42:517-525), the degeneration is very rapid and in this regard differs from the usual, slower time course observed in the human disease. In this strain, photoreceptor cell degeneration begins around P8, a time when the retinal vasculature is still rapidly expanding (FIG. 15). Subsequent degeneration of the deep retinal vasculature occurs even while the intermediate plexus is still forming and, thus, the retinas of *rd1*/*rd1* mice never completely develops, unlike that observed in most humans with this disease. An *rd10* mouse model, which has a slower time course of degeneration and more closely resembles the human retinal degenerative condition, was used to investigate Lin⁻ HSC-mediated vascular rescue. In the *rd10* mouse, photoreceptor cell degeneration begins around P21 and vascular degeneration begins shortly thereafter.

Since normal neurosensory retinal development is largely complete by P21, the degeneration is observed to start after the retina has completed differentiation and in this way is more analogous to human retinal degenerations than the *rd1*/*rd1* mouse model. Lin⁻ HSCs or control cells from *rd10* mice were injected into P6 eyes and the retinas were evaluated at varying time points. At P21 the retinas from both Lin⁻ HSC and control cell-injected eyes appeared normal with complete development of all vascular layers and normal development of the INL and ONL (FIG. 18 (D and H)). At approximately P21 the retinal degeneration began and progressed with age. By P30, the control cell-injected retinas exhibited severe vascular and neuronal degeneration (FIG. 18 (I)), while the Lin⁻HSC-injected retinas maintained nearly normal vascular layers and photoreceptor cells (FIG. 18 (E)). The difference between the rescued and non-rescued eyes was more pronounced at later time points (compare FIG. 18 (F and G) to 18 (J and K)). In the control treated eyes, the progression of vascular degeneration was very clearly observed by immunohistochemical staining for CD31 and collagen IV (FIG. 18 (I-K)). The control-treated eyes were nearly completely negative for CD31, whereas collagen IV-positive vascular "tracks" remained evident, indicating that vascular regression, rather than incomplete vascular formation, had occurred. In contrast, Lin⁻ HSC-treated eyes had both CD31 and collagen IV-positive vessels that appeared very similar to normal, wild-type eyes (compare FIG. 18 (F and I)).

### Gene Expression Analysis of rd/rd Mouse Retinas after Lin⁻ HSC Treatment.

Large scale genomics (microarray analysis) was used to analyze rescued and non-rescued retinas to identify putative mediators of neurotrophic rescue. Gene expression in *rd1*/*rd1* mouse retinas treated with Lin⁻ HSCs was compared to uninjected retinas as well as retinas injected with control cells (CD31⁻). These comparisons each were performed in triplicate. To be considered present, genes were required to have expression levels at least 2-fold higher than background levels in all three triplicates. Genes that were upregulated 3-fold in Lin⁻ HSC-protected retinas compared to control cell-injected and non-injected *rd*/*rd* mouse retinas are shown in FIG. 20, panels A and B. Coefficient of variance (COV) levels were calculated for the expressed genes by dividing the standard deviation by the mean expression level of each cRNA replicate. In addition, the correlation between expression levels and noise variance was calculated by correlating the mean and standard deviation (SD). A correlation between gene expression level and standard deviation for each gene was obtained, allowing background levels and reliable expression level thresholds to be determined. As a whole, the data fell well within acceptable limits (Tu et al. 2002, Proc. Natl. Acad. Sci. U S A 99: 14031-14036). The genes that are discussed individually, below, exhibited expression levels above these critical expression levels. Paired "t-test" values for the discussed genes were also determined. In each case, *p*-values are reasonable (near or below 0.05), which demonstrates that there are similarities between replicates and probable significant differences between the different test groups. Many of the significantly upregulated genes, including MAD and Ying Yang-1 (YY-1) (Austen et al. 1997, Curr. Top. Microbiol. Immunol. 224: 123-130.), encode proteins with functions involving the protection of cells from apoptosis. A number of crystallin genes, which have sequence homology and similar functions to known heat-shock proteins involving protection of cells from stress, were also upregulated by Lin- HSC treatment. Expression of α-crystallin was localized to the ONL by immunohistochemical analysis (FIG. 19). FIG. 19 shows that crystallin αA is upregulated in rescued outer nuclear layer cells after treatment with Lin⁻HSCs but not in contralateral eyes treated with control cells. The left panel shows IgG staining (control) in rescued retina. The middle panel shows crystallin αA in a rescued retina. The right panel shows crystallin αA in non-rescued retina.

Messenger RNA from *rd1*/*rd1* mouse retinas rescued with human Lin⁻ HSCs were hybridized to human specific Affymetrix U133A microarray chips. After stringent analysis, a number of genes were found whose mRNA expression was human specific, above background, and significantly higher in the human Lin⁻ HSC rescued retinas compared to the murine Lin⁻ HSC rescued retinas and the human control cell-injected non-rescued retinas (FIG. 20, panel C). CD6, a cell adhesion molecule expressed at the surface of primitive and newly differentiated CD34+ hematopoietic stem cells, and interferon alpha 13, another gene expressed by hematopoietic stem cells, were both found by the microarray bioinformatics technique, validating the evaluation protocol. In addition, several growth factors and neurotrophic factors were expressed above background by human Lin⁻ HSC rescued mouse retina samples (FIG. 20, panel D).

Markers for lineage-committed hematopoietic cells were used to negatively select a population of bone marrow-derived Lin⁻ HSC containing EPC. While the sub-population of bone marrow-derived Lin⁻ HSC that can serve as EPC is not characterized by commonly used cell surface markers, the behavior of these cells in developing or injured retinal vasculature is entirely different than that observed for Lin⁺ or adult endothelial cell populations. These cells selectively target to sites of retinal angiogenesis and participate in the formation of patent blood vessels.

Inherited retinal degenerative diseases are often accompanied by loss of retinal vasculature. Effective treatment of such diseases requires restoration of function as well as maintenance of complex tissue architecture. While several recent studies have explored the use of cell-based delivery of trophic factors or stem cells themselves, some combination of both may be necessary. For example, use of growth factor therapy to treat retinal degenerative disease resulted in unregulated overgrowth of blood vessels resulting in severe disruption of the normal retinal tissue architecture. The use of neural or retinal stem cells to treat retinal degenerative disease may reconstitute neuronal function, but a functional vasculature will also be necessary to maintain retinal functional integrity. Incorporation of cells from a Lin⁻HSC population into the retinal vessels of *rd*/*rd* mice stabilized the degenerative vasculature without disrupting retinal structure. This rescue effect was also observed when the cells were injected into P15 *rd*/*rd* mice. Since vascular degeneration begins on P16 in *rd*/*rd* mice, this observation expands the therapeutic window for effective Lin⁻ HSC treatment. Retinal neurons and photoreceptors are preserved and visual function is maintained in eyes injected with the Lin⁻ HSC cells.

Adult bone marrow-derived Lin⁻HSCs exert profound vasculo- and neurotrophic effects when injected intravitreally into mice with retinal degenerative disease. This rescue effect persists for up to 6 months after treatment and is most efficacious when the Lin⁻HSCs are injected prior to complete retinal degeneration (up to 16 days after birth in mice that ordinarily exhibit complete retinal degeneration by 30 days postnatally). This rescue is observed in two mouse models of retinal degeneration and, remarkably, can be accomplished with adult human bone marrow-derived HSCs when the recipient is an immunodeficient rodent with retinal degeneration (e.g., the SCID mouse) or when the donor is a mouse with retinal degeneration. While several recent reports have described a partial phenotypic rescue in mice or dogs with retinal degeneration after viral based gene rescue with the wild type gene (Ali, et al. 2000, Nat Genet 25:306-310; Takahashi et al. 1999, J. Virol. 73:7812-7816; Acland et al. 2001, Nat. Genet. 28:92-95.), the present invention is the first generic cell-based therapeutic effect achieved by vascular rescue. Thus, the potential utility of such an approach in treating a group of diseases (e.g., retinitis pigmentosa) with over 100 known associated mutations is more practical than creating individual gene therapies to treat each known mutation.

The precise molecular basis of the neurotrophic rescue effect remains unknown, but is observed only when there is concomitant vascular stabilization/rescue. The presence of injected stem cells, per se, is not sufficient to generate a neurotrophic rescue and the clear absence of stem cell-derived neurons in the outer nuclear layer rules out the possibility that the injected cells are transforming into photoreceptors. Data obtained by microarray gene expression analysis demonstrated a significant up-regulation of genes known to have anti-apoptotic effects. Since most neuronal death observed in retinal degenerations is by apoptosis, such protection may be of great therapeutic benefit in prolonging the life of photoreceptors and other neurons critical to visual function in these diseases. C-myc is a transcription factor that participates in apoptosis by upregulation of various downstream apoptosis-inducing factors. C-myc expression was increased 4.5 fold in *rd*/*rd* mice over wild-type indicating potential involvement in the photoreceptor degeneration observed in the *rd1*/*rd1* mouse. Mad1 and YY-1, two genes dramatically upregulated in Lin⁻HSC-protected retinas (FIG. 20, panel A), are known to suppress the activity of c-myc, thus inhibiting c-myc induced apoptosis. Overexpression of Mad1 has also been shown to suppress Fas-induced activation of caspase-8, another critical component of the apoptotic pathway. Upregulation of these two molecules may play a role in protection of the retina from vascular and neural degeneration by preventing the initiation of apoptosis that normally leads to degeneration in *rd*/*rd* mice.

Another set of genes that were greatly upregulated in Lin⁻HSC protected retinas includes members of the crystallin family (FIG. 20, panel B). Similar to heat-shock and other stress-induced proteins, crystallins may be activated by retinal stress and provide a protective effect against apoptosis. Abnormally low expression of crystallin αA is correlated with photoreceptor loss in a rat model of retinal dystrophy and a recent proteomic analysis of the retina in the *rd*/*rd* mouse demonstrated induction of crystallin upregulation in response to retinal degeneration. Based on our microarray data of EPC-rescued *rd*/*rd* mouse retinas, upregulation of crystallins appear to play a key role in EPC mediated retinal neuroprotection.

Genes such as c-myc, Mad1, Yx-1 and the crystallins are likely to be downstream mediators of neuronal rescue. Neurotrophic agents can regulate anti-apoptotic gene expression, although our microarray analysis of retinas rescued with mouse stem cells did not demonstrate induction of increased levels of known neurotrophic factors. Analysis of human bone marrow-derived stem cell-mediated rescue with human specific chips did, on the other hand, demonstrate low, but significant increases in the expression of multiple growth factor genes.

The upregulated genes include several members of the fibroblast growth factor family and otoferlin. Mutations in the otoferlin gene are associated with genetic disorders leading to deafness due to auditory neuropathy. It is possible that otoferlin production by injected Lin⁻HSCs contributes to the prevention of retinal neuropathy as well. Historically, it has long been assumed that vascular changes observed in patients and animals with retinal degeneration were secondary to decreased metabolic demand as the photoreceptors die. The present data indicate that, at least for mice with inherited retinal degeneration, preserving normal vasculature can help maintain components of the outer nuclear layer as well. Recent reports in the literature would support the concept that tissue-specific vasculature has trophic effects that go beyond that expected from simply providing vascular "nourishment." For example, liver endothelial cells can be induced to produce, after VEGFR1 activation, growth factors critical to hepatocyte regeneration and maintenance in the face of hepatic injury (LeCouter et al. 2003, Science 299:890-893).

Similar indicative interactions between vascular endothelial cells and adjacent hepatic parenchymal cells are reportedly involved in liver organogenesis, well before the formation of functional blood vessels. Endogenous retinal vasculature in individuals with retinal degeneration may not facilitate so dramatic a rescue, but if this vasculature is buttressed with endothelial progenitors derived from bone marrow hematopoietic stem cell populations, they may make the vasculature more resistant to degeneration and at the same time facilitate retinal neuronal, as well as vascular, survival. In humans with retinal degeneration, delaying the onset of complete retinal degeneration may provide years of additional sight. The animals treated with Lin⁻HSCs had significant preservation of an ERG, which may be sufficient to support vision.

Clinically, it is widely appreciated that there may be substantial loss of photoreceptors and other neurons while still preserving functional vision. At some point, the critical threshold is crossed and vision is lost. Since nearly all of the human inherited retinal degenerations are of early, but slow, onset, an individual with retinal degeneration can be identified and treated intravitreally with a graft of autologous bone marrow stem cells to delay retinal degeneration and concomitant loss of vision. To enhance targeting and incorporation of cells , the presence of activated astrocytes is desirable (Otani et al. 2002, Nat. Med. 8: 1004-1010); this can be accomplished by early treatment when there is an associated gliosis, or by using a laser to stimulate local proliferation of activated astrocytes. Optionally, *ex vivo* transfection of the stem cells with one or more neurotrophic substances prior to intraocular injection can be used to enhance the rescue effect. This approach can be applied to the treatment of other visual neuronal degenerative disorders, such as glaucoma, in which there is retinal ganglion cell degeneration.

The Lin⁻ HSC populations from adult bone marrow contain a population of EPC that can promote angiogenesis by targeting reactive astrocytes and incorporate into an established template without disrupting retinal structure. The Lin⁻ HSC also provide a long-term neurotrophic rescue effect in eyes suffering from retinal degeneration. In addition, genetically modified, autologous Lin⁻ HSC compositions containing EPC can be transplanted into ischemic or abnormally vascularized eyes and can stably incorporate into new vessels and neuronal layers and continuously deliver therapeutic molecules locally for prolonged periods of time. Such local delivery of genes that express pharmacological agents in physiologically meaningful doses represents a new paradigm for treating currently untreatable ocular diseases.

Photoreceptors in the normal mouse retina, for example, are predominantly rods, but the outer nuclear layer observed after rescue with Lin⁻HSCs contained predominantly cones. Most inherited human retinal degenerations occur as a result of primary rod-specific defects, and loss of the cones is believed to be secondary to rod dysfunction, which is likely related to the loss of some trophic factor expressed by rods.

### EXAMPLES

### Example 1. Cell Isolation and Enrichment; Preparation of Murine Lin⁻ HSC Populations A and B.

**General Procedure.** All *in vivo* evaluations were performed in accordance with the NIH Guide for the Care and Use of Laboratory Animals, and all evaluation procedures were approved by The Scripps Research Institute (TSRI, La Jolla, CA) Animal Care and Use Committee. Bone marrow cells were extracted from B6.129S7-Gtrosa26, Tie-2GFP, ACTbEGFP, FVB/NJ (*rd*/*rd* mice) or Balb/cBYJ adult mice (The Jackson Laboratory, ME).

Monocytes were then separated by density gradient separation using HISTOPAQLTE® polysucrose gradient (Sigma, St. Louis, MO) and labeled with biotin conjugated lineage panel antibodies (CD45, CD3, Ly-6G, CD11, TER-119, Pharmingen, San Diego, CA) for Lin⁻ selection in mice. Lineage positive (Lin⁺) cells were separated and removed from Lin⁻ HSC using a magnetic separation device (AUTOMACS™ sorter, Miltenyi Biotech, Auburn, CA). The resulting Lin⁻ HSC population, containing endothelial progenitor cells was further characterized using a FACS™ Calibur flow cytometer (Becton Dickinson, Franklin Lakes, NJ) using the following antibodies: PE-conjugated-Sca-1, c-kit, KDR, and CD31 (Pharmingen, San Diego, CA). Tie-2-GFP bone marrow cells were used for the characterization of Tie-2.

To harvest adult mouse endothelial cells, mesenteric tissue was surgically removed from ACTbEGFP mouse and placed in collagenase (Worthington, Lakewood, NJ) to digest the tissue, followed by filtration using a 45µm filter. Flow-through was collected and incubated with Endothelial Growth Media (Clonetics, San Diego, CA). Endothelial characteristics were confirmed by observing morphological cobblestone appearance, staining with CD31 mAb (Pharmingen) and examining cultures for the formation of tube-like structures in MATRIGEL™ matrix (Beckton Dickinson, Franklin Lakes, NJ).

**Murine Lin⁻ HSC Population A.** Bone marrow cells were extracted from ACTbEGFP mice by the General Procedure described above. The Lin⁻ HSC cells were characterized by FACS flow cytometry for CD31, c-kit, Sca-1, Flk-1, and Tie-2 cell surface antigen markers. The results are shown in FIG. 1 (c). About 81% of the Lin⁻ HSC exhibited the CD31 marker, about 70.5% of the Lin⁻ HSC exhibited the c-kit marker, about 4% of the Lin⁻ HSC exhibited the Sca-1 marker, about 2.2% of the Lin⁻ HSC exhibited the Flk-1 marker and about 0.91% of the Lin⁻ HSC cell exhibited the Tie-2 marker. In contrast, the Lin⁺ HSC that were isolated from these bone marrow cells had a significantly different cell marker profile (i.e., CD31: 37.4%; c-kit: 20%; Sca-1: 2.8%; Flk-: 0.05%).

**Murine Lin⁻ HSC Population B.** Bone marrow cells were extracted from Balb/C, ACTbEGFP, and C3H mice by the General Procedure described above. The Lin⁻ HSC cells were analyzed for the presence of cell surface markers (Sca-1, Flk-1/KDR, c-kit (CD117), CD34, CD31 and various integrins: α1, α2, α3, α4, a5, α6, α_{L}, α_{M} α_{V}, α_{X}, α_{IIb},, β₁, β₂, β₃, β₄, β₅ and β₇). The results are shown in Table 2.

**Table 2. Characterization of Lin⁻ HSC Population B.**

| **Cell Marker** | **Lin⁻ HSC** |
|---|---|
| α1 | 0.10 |
| α2 | 17.57 |
| α3 | 0.22 |
| α4 | 89.39 |
| α5 | 82.47 |
| α6 | 77.70 |
| αL | 62.69 |
| αM | 35.84 |
| αX | 3.98 |
| αV | 33.64 |
| αIIb | 0.25 |
| β1 | 86.26 |
| β2 | 49.07 |
| β3 | 45.70 |
| β4 | 0.68 |
| β5 | 9.44 |
| β7 | 11.25 |
| CD31 | 51.76 |
| CD34 | 55.83 |
| Flk-1/KDR | 2.95 |
| c-kit (CD117) | 74.42 |
| Sca-1 | 7.54 |

### Example 2. Intravitreal Administration of Cells in a Murine Model.

An eyelid fissure was created in a mouse eyelid with a fine blade to expose the P2 to P6 eyeball. Lineage negative HSC Population A (approximately 10⁵ cells in about 0.5 µl to about 1 µl of cell culture medium) was then injected intravitreally using a 33-gauge (Hamilton, Reno, NV) needled-syringe.

### Example 3. EPC Transfection.

Murine Lin⁻ HSC (Population A) were transfected with DNA (SEQ ID NO: 1, FIG. 7) encoding the T2 fragment of TrpRS (SEQ ID NO: 3) also enclosing a His₆ tag using FuGENE™6 Transfection Reagent (Roche, Indianapolis, IN) according to manufacturer's protocol. Lin⁻ HSC cells (about 10⁶ cell per ml) were suspended in opti-MEM® medium (Invitrogen, Carlsbad, CA) containing stem cell factor (PeproTech, Rocky Hill, NJ). DNA (about 1 µg) and FuGENE reagent (about 3 µl) mixture was then added, and the mixtures were incubated at about 37°C for about 18 hours. After incubation, cells were washed and collected. The transfection rate of this system was approximately 17% as confirmed by FACS analysis. T2-TrpRS production was confirmed by western blotting. The amino acid sequence of His₆-tagged T2-TrpRS is shown as SEQ ID NO: 2, FIG. 8.

### Example 4. Immunohistochemistry and Confocal Analysis.

Mouse retinas were harvested at various time points and were prepared for either whole mounting or frozen sectioning. For whole mounts, retinas were fixed with 4% paraformaldehyde, and blocked in 50% fetal bovine serum (FBS) and 20% normal goat serum for one hour at ambient room temperature. Retinas were processed for primary antibodies and detected with secondary antibodies. The primaries used were: anti-Collagen IV (Chemicon, Temecula, CA, anti-β-gal (Promega, Madison, WI), anti-GFAP (Dako Cytomation, Carpenteria, CA), anti-α-smooth muscle actin (α-SMA, Dako Cytomation). Secondary antibodies used were conjugated either to Alexa 488 or 594 fluorescent markers (Molecular Probes, Eugene, OR). Images were taken using an MRC 1024 Confocal microscope (Bio-Rad, Hercules, CA). Three-dimensional images were created using LASERSHARP® software (Bio-Rad) to examine the three different layers of vascular development in the whole mount retina. The difference in GFP pixel intensity between enhanced GFP (eGFP) mice and GFAP/wtGFP mice, distinguished by confocal microscopy, was utilized to create the 3 dimensional images.

### Example 5. In vivo Retinal Angiogenesis Quantification Assay in Mice.

For T2-TrpRS analysis, the primary and deep plexus were reconstructed from the three dimensional images of mouse retinas. The primary plexus was divided into two categories: normal development, or halted vascular progression. The categories of inhibition of deep vascular development were construed based upon the percentage of vascular inhibition including the following criteria: complete inhibition of deep plexus formation was labeled "Complete", normal vascular development (including less than 25% inhibition) was labeled "Normal" and the remainder labeled "Partial." For the *rd*/*rd* mouse rescue data, four separate areas of the deeper plexus in each whole mounted retina was captured using a 10x lens. The total length of vasculature was calculated for each image, summarized and compared between the groups. To acquire accurate information, Lin⁻ HSC were injected into one eye and Lin⁺ HSC into another eye of the same mouse. Non-injected control retinas were taken from the same litter.

### Example 6. Adult Retinal Injury Murine Models.

Laser and scar models were created using either a diode laser (150 mW, 1 second, 50 mm) or mechanically by puncturing the mouse retina with a 27 gauge needle. Five days after injury, cells were injected using the intravitreal method. Eyes were harvested from the mice five days later.

### Example 7. Neurotrophic Rescue of Retinal Regeneration.

Adult murine bone marrow derived lineage negative hematopoietic stem cells (Lin⁻ HSC) have a vasculotrophic and neurotrophic rescue effect in a mouse model of retinal degeneration. Right eyes of 10-day old mice were injected intravitreally with about 0.5 microliters containing about 10⁵ Lin-HSC and evaluated 2 months later for the presence of retinal vasculature and neuronal layer nuclear count. The left eyes of the same mice were injected with about the same number of Lin⁺HSC as a control, and were similarly evaluated. As shown in FIG. 9, in the Lin⁻HSC treated eyes, the retinal vasculature appeared nearly normal, the inner nuclear layer was nearly normal and the outer nuclear layer (ONL) had about 3 to about 4 layers of nuclei. In contrast, the contralateral Lin⁺HSC treated eye had a markedly atrophic middle retinal vascular layer, a completely atrophic outer retinal vascular layer; the inner nuclear layer was markedly atrophic and the outer nuclear layer was completely gone. This was dramatically illustrated in Mouse 3 and Mouse 5. In Mouse 1, there was no rescue effect and this was true for approximately 15% of the injected mice.

When visual function was assessed with electroretinograms (ERG), the restoration of a positive ERG was observed when both the vascular and neuronal rescue was observed (Mice 3 and 5). Positive ERG was not observed when there was no vascular or neuronal rescue (Mouse 1). This correlation between vascular and neurotrophic rescue of the rd/rd mouse eyes by the Lin⁻HSC is illustrated by a regression analysis plot shown in FIG. 10. A correlation between neuronal (y-axis) and vascular (x-axis) recovery was observed for the intermediate vasculature type (r=0.45) and for the deep vasculature (r=0.67).

FIG. 11 shows the absence of any statistically significant correlation between vascular and neuronal rescue by Lin⁺ HSC. The vascular rescue was quantified and the data are presented in FIG. 12. Data for mice at 1 month (1M), 2 months (2M), and 6 months (6M), post-injection shown in FIG. 12, demonstrate that vascular length was significantly increased in eyes treated with the Lin⁻HSC (dark bars) relative to the vascular length in untreated eyes from the same mouse (light bars), particularly at 1 month and 2 months, post-injection. The neurotrophic rescue effect was quantified by counting nuclei in the inner and outer nuclear layers about two months after injection of Lin⁻ HSC or Lin⁺HSC. The results are presented in FIG. 13 and 14.

### Example 8. Human Lin⁻ HSC Population.

Bone marrow cells were extracted from healthy adult human volunteers by the General Procedure described above. Monocytes were then separated by density gradient separation using HISTOPAQUE® polysucrose gradient (Sigma, St. Louis, MO). To isolate the Lin⁻ HSC population from human bone marrow mononuclear cells the following biotin conjugated lineage panel antibodies were used with the magnetic separation system (AUTOMACS^{™} sorter, Miltenyi Biotech, Auburn, CA): CD2, CD3, CD4, CD11a, Mac-1, CD14, CD16, CD19, CD33, CD38, CD45RA, CD64, CD68, CD86, CD235a (Pharmingen).

The human Lin⁻ HSC population was further separated into two sub-populations based on CD133 expression. The cells were labeled with biotin-conjugated CD133 antibodies and separated into CD133 positive and CD133 negative sub-populations.

### Example 9. Intravitreal Administration of Human and Murine Cells in Murine Models for Retinal Degeneration.

C3H/HeJ, C3SnSmn.CB17-*Prkdc SCID,* and *rd10* mouse strains were used as retinal degeneration models. C3H/HeJ and C3SnSmn.CB17-*Prkdc SCID* mice (The Jackson Laboratory, Maine) were homozygous for the retinal degeneration 1 (*rd1*) mutation, a mutation that causes early onset severe retinal degeneration. The mutation is located in exon 7 of the *Pde6b* gene encoding the rod photoreceptor cGMP phosphodiesterase P subunit. The mutation in this gene has been found in human patients with autosomal recessive retinitis pigmentosa (RP). C3SnSmn.CB17-*Prkdc SCID* mice are also homozygous for the severe combined immune deficiency spontaneous mutation (*Prkdc SCID*) and were used for human cell transfer experiments. Retinal degeneration in *rd10* mice is caused by a mutation in exon 13 of *Pde6b* gene. This is also a clinically relevant RP model with later onset and milder retinal degeneration than *rd1*/*rd1).* All evaluations were performed in accordance with the NIH Guide for the Care and Use of Laboratory Animals, and all procedures were approved by The Scripps Research Institute Animal Care and Use Committee.

An eyelid fissure was created in a mouse eyelid with a fine blade to expose the P2 to P6 eyeball. Lineage negative HSC cells for murine population A or human population C (approximately 10⁵ cells in about 0.5 µl to about 1 µl of cell culture medium) were then injected in the mouse eye intravitreally using a 33-gauge (Hamilton, Reno, NV) needled-syringe. To visualize the injected human cells, cells were labeled with dye (Cell tracker green CMFDA, Molecular Probes) before injection.

Retinas were harvested at various time points and fixed with 4% paraformaldehyde (PFA) and methanol followed by blocking in 50% FBS/20% NGS for one hour at room temperature. To stain retinal vasculature, retinas were incubated with anti-CD31 (Pharmingen) and anti-collagen IV (Chemicon) antibodies followed by Alexa 488 or 594 conjugated secondary antibodies (Molecular Probes, Eugene, Oregon). The retinas were laid flat with four radial relaxing incisions to obtain a whole mount preparation. Images of vasculature in intermediate or deep retinal vascular plexuses (see Dorrell et al. 2002 Invest Ophthalmol. Vis. Sci. 43:3500-3510) were obtained using a Radiance MP2100 confocal microscope and LASERSHARP® software (Biorad, Hercules, California). For quantification of vasculature, four independent fields (900 µm x 900 µm) were chosen randomly from the mid portion of the intermediate or deep vascular layer and the total length of vasculature was measured using LASERPIX® analyzing software (Biorad). The total lengths of these four fields in the same plexus were used for further analysis.

The flat-mounted retinas were re-embedded for cryostat sections. Retinas were placed in 4% PFA overnight followed by incubation with 20% sucrose. The retinas were embedded in optimal cutting temperature compound (OCT: Tissue-Tek; Sakura FineTech, Torrance, CA). Cryostat sections (10 µm) were re-hydrated in PBS containing the nuclear dye DAPI (Sigma-Aldrich, St. Louis, Missouri). DAPI-labeled nuclear images of three different areas (280 µm width, unbiased sampling) in a single section that contained optic nerve head and the entire peripheral retina were taken by confocal microscope. The numbers of the nuclei located in ONL of the three independent fields in one section were counted and summed up for analysis. Simple linear-regression analysis was performed to examine the relationship between the length of vasculature in the deep plexus and the number of cell nuclei in the ONL.

Following overnight dark-adaptation, mice were anesthetized by intraperitoneal injection of 15 µg/gm ketamine and 7 µg/gm xylazine. Electroretinograms (ERGs) were recorded from the corneal surface of each eye after pupil dilation (1% atropine sulfate) using a gold loop corneal electrode together with a mouth reference and tail ground electrode. Stimuli were produced with a Grass Photic Stimulator (PS33 Plus, Grass Instruments, Quincy, MA) affixed to the outside of a highly reflective Ganzfeld dome. Rod responses were recorded to short-wavelength (Wratten 47A; λₘₐₓ = 470 nm) flashes of light over a range of intensities up to the maximum allowable by the photic stimulator (0.668 cd-s/m²). Response signals were amplified (CP511 AC amplifier, Grass Instruments), digitized (PCI-1200, National Instruments, Austin, TX) and computer-analyzed. Each mouse served as its own internal control with ERGs recorded from both the treated and untreated eyes. Up to 100 sweeps were averaged for the weakest signals. The averaged responses from the untreated eye were digitally subtracted from the responses from the treated eye and this difference in signal was used to index functional rescue.

Microarray analysis was used for evaluation of Lin⁻ HSC-targeted retinal gene expression. P6 *r*d/*rd* mice were injected with either Lin⁻ or CD31⁻ HSCs. The retinas of these mice were dissected 40 days post-injection in RNase free medium (rescue of the retinal vasculature and the photoreceptor layer is obvious at this time point after injection). One quadrant from each retina was analyzed by whole mount to ensure that normal HSC targeting as well as vasculature and neural protection had been achieved. RNA from retinas with successful injections was purified using a TRIzol (Life Technologies, Rockville, MD), phenol/chloroform RNA isolation protocol. RNA was hybridized to Affymetrix Mu74Av2 chips and gene expression was analyzed using GENESPRING® software (SiliconGenetics, Redwood City, CA). Purified human or mouse HSCs were injected intravitreally into P6 mice. At P45 the retinas were dissected and pooled into fractions of 1) human HSC-injected, rescued mouse retinas, 2) human HSC-injected, non-rescued mouse retinas, and 3) mouse HSC-injected, rescued mouse retinas for purification of RNA and hybridization to human-specific U133A Affymetrix chips. GENESPRING® software was used to identify genes that were expressed above background and with higher expression in the human HSC-rescued retinas. The probe-pair expression profiles for each of these genes were then individually analyzed and compared to a model of normal human U133A microarray experiments using dChip to determine human species specific hybridization and to eliminate false positives due to cross-species hybridization.

FIG. 21 illustrates flow cytometry data comparing the expression of CD31 and integrin alpha 6 surface antigens on CD133 positive (CD133⁺) and CD133 negative (CD133⁻) human Lin⁻ HSC populations. The left panels show flow cytometry scatter plots. The center and right panels are histograms showing the level of specific antibody expression on the cell population. The Y axis represents the number of events and the X axis shows the intensity of the signal. The outlined histograms are isotype IgG control antibodies showing the level of non-specific background staining. The filled histograms show the level of specific antibody expression on the cell population. A filled histogram shifted to the right of the outlined (control) histogram represents an increased fluorescent signal and expression of the antibody above background level. Comparing the position of the peaks of the filled histograms between the two cell populations represents the difference in protein expression on the cells. For example, CD31 is expressed above background on both CD133⁺ and CD133⁻ cells; however, there are more cells expressing lower levels of CD31 in the CD133⁺ cell population than there are in the CD133- population. From this data it is evident that CD31 expression varies between the two populations and that the alpha 6 integrin expression is largely limited to cells in the Lin⁻ population, and thus may serve as a marker of cells with vasculo- and neurotrophic rescue function.

When the CD133 positive and CD133 negative Lin⁻ HSC sub-population was intravitreally injected into the eyes of neonatal SCID mice, the greatest extent of incorporation into the developing vasculature was observed for the CD133 negative sub-population, which expresses both CD31 and integrin α6 surface antigens (see FIG. 21, bottom). The CD133 positive sub-population, which does not express CD31 or integrin α6 (FIG. 21, top) appears to target sites of peripheral ischemia-driven neovascularization, but not when injected into eyes undergoing angiogenesis.

Rescued and non-rescued retinas were analyzed immunohistochemically with antibodies specific for rod or cone opsin. The same eyes used for the ERG recordings presented in FIG. 17 were analyzed for rod or cone opsin. In wild type mouse retinas, less than 5% of photoreceptors present are cones (Soucy et al. 1998, Neuron 21: 481-493) and the immunohistochemical staining patterns observed with red/green cone opsin as shown in FIG. 25 (A) or rod rhodopsin as shown in FIG. 25 (B), were consistent with this percentage of cone cells. Antibodies specific for rod rhodopsin (rho4D2) were provided by Dr. Robert Molday of the University of British Columbia and used as described previously (Hicks et al. 1986, Exp. Eye Res. 42: 55-71). Rabbit antibodies specific for cone red/green opsin were purchased from Chemicon (AB5405) and used according to the manufacturer's instructions.

### Example 10. Intravitreal Administration of Murine Cells in Murine Models for Oxygen Induced Retinal Degeneration.

New born wild-type C57B16 mice were exposed to hyperoxia (75% oxygen) between postnatal days P7 to P12 in an oxygen-induced retinal degeneration (OIR) model. FIG 22 illustrates normal postnatal vascular development in C57B 16 mice from P0 to P30. At P0 only budding superficial vessels can be observed around the optic disc. Over the next few days, the primary superficial network extends toward the periphery, reaching the far periphery by day P10. Between P7 and P12, the secondary (deep) plexus develops. By P17, an extensive superficial and deep network of vessels is present (FIG. 22, insets). In the ensuing days, remodeling occurs along with development of the tertiary (intermediate) layer of vessels until the adult structure is reached approximately at P21.

In contrast, in the OIR model described herein, following exposure to 75% oxygen at P7-P12, the normal sequence of events is severely disrupted (FIG. 23). Adult murine Lin⁻ HSC populations were intravitreally injected at P3 in an eye of a mouse that was subsequently subjected to OIR, the other eye was injected with PBS or CD31 negative cells as a control. FIG. 24 illustrates that the Lin⁻ HSC populations can reverse the degenerative effects of high oxygen levels in the developing mouse retina. Fully developed superficial and deep retinal vasculature was observed at P 17 in the treated eyes, whereas the control eyes showed large avascular areas with virtually no deep vessels (FIG. 24). Approximately 100 eyes of mice in the OIR model were observed. Normal vascularization was observed in 58% of the eyes treated with the Lin⁻ HSC populations, compared to 12% of the control eyes treated with CD31- cells and 3% of the control eyes treated with PBS.

### Example 11. Isolation of Myeloid-Like Bone Marrow Cells From Murine Bone Marrow by CD44 Selection.

Bone marrow cells were extracted from adult mice (The Jackson Laboratory, ME). The whole bone marrow was treated with a murine CD44 antibody and flow cytometry was used to isolate CD44 expressing cells from the bone marrow. The cells were separated from the antibody and stored in a buffer solution for future use. A population of cells that do not significantly express CD44 was also isolated (CD44^{lo}BM).

### Example 12. Isolation of Myeloid-Like Bone Marrow Cells From Murine Bone Marrow by CD44 Selection.

Bone marrow cells were also positively selected using an antibody to CD11b in place of CD44, as described in Example 11. A myeloid-like bone marrow cell population that was CD44^{hi} and CD11b+ was isolated, which had similar activity characteristics to the CD44^{hi} population isolated in Example 11 using CD44. A CD44^{lo} CD11b⁻ population was also isolated, which was found to be inactive.

### Example 13. Characterization of the MLBM Cell Populations.

Although the role of CD44 in this context is not clear, it is possible that this receptor mediates cell survival, cell migration and/or cell differentiation in the hyaluronic acid-rich vitreous following injection of cells into the eye. Distinct populations of CD44^{hi} (i.e., MLBM) and CD44^{lo} cells were present in unfractionated mouse bone marrow. The MLBM cell population represents 76% of the Lin⁻ population used in previous examples, whereas only about 37% and 4%, respectively, of Lin⁺ and CD31⁻/CD34⁻/CD11b⁻ cell populations from bone marrow expressed CD44 (FIG. 26). Accordingly, there is an excellent correlation between CD44 expression and the vasculotrophic and neurotrophic activities observed in these three populations, i.e. Lin⁻ cells were the most effective while CD31-/CD34-/CD11b⁻ cells were consistently the least effective. Using a panel of lineage-specific antibodies, the majority of CD44^{hi} cells were determined to have strongly myeloid characteristics (FIG. 27). Similarly, nearly all of the CD44^{hi} bone marrow cells are also CD11b⁺ (FIG. 27).

MLBM positively selected using CD11b antibody in Example 12 (CD44^{hi} CD11b⁺) gave activity results similar to those obtained with MLBM isolated using CD44 antibody selection in the vascular targeting experiments.

The cell surface antigen characteristics of the MLBM cell population of Example 12 and of the CD44^{lo} CD11b+ cells isolated in Example 12 are shown in Table 3, below. In Table 3, a greater number of plus signs (+) indicates relatively higher expression of the antigen. A minus sign (-) indicates no expression detected.

**Table 3**

| **Antigen** | **CD44^{hi}/CD11b+** | **CD44^{lo}/CD11b-** |
|---|---|---|
| CD11a | +++ | + |
| CD31 | + | ++ |
| CD34 | + | - |
| alpha 6 | ++ | - |
| KDR | + | - |
| Sca-1 | + | + |
| c-Kit | + | - |
| CD115 | + | - |
| CD45RB220 | + | ++ |
| TER119 | - | +++ |
| Ly6G&C (GR-1) | +++ | - |
| Ly6G | +++ | - |

### Example 14. Vasculotrophic and Neurotrophic Effects of The MLBM Cell Population.

The MLBM cell population of Example 11 retained the properties of Lin⁻ cells in terms of vascular targeting and vasculo- and neurotrophic effects, while CD44^{lo}BM cells showed little or no activity. Vascular targeting activity was demonstrated by injecting cells from a GFP⁺ MLBM cell population intravitreally into postnatal day 7 (P7) mice and analyzing retinas at P14. After labeling blood vessels with GS isolectin, GFP⁺ cells were observed to target the retinal vasculature and assume a perivascular localization, without evidence of incorporation. These events were common when using MLBM, but infrequent or absent in eyes treated with CD44^{lo}BM (FIG. 28).

Vasculo- and neurotrophic activity of the MLBM cell population of Example 11 was evaluated using a mouse model of retinal degeneration as described above for Lin⁻ HSC. The *rd1*/*rd1* mouse shows characteristic features of retinal degenerative disease including photoreceptor death and atrophy of the deep retinal vasculature. As described above, Lin⁻ HSC bone marrow cells preserved the deep retinal vasculature and partially rescued photoreceptors. The MLBM cell population performs the same function (FIG. 29).

The oxygen-induced retinopathy model shares features with retinopathy of prematurity. The pathology associated with this model is significantly reduced when eyes are treated with cells from the MLBM cell population. The effects of cells from the MLBM cell population in this model were similar to those observed using Lin⁻HSCs described above. Eyes treated with cells from the MLBM cell population showed significant reduction in the two parameters used to quantify the degree of pathology in this model: vascular obliteration area and neovascular tuft area. In contrast, eyes treated with CD44¹⁰BM cells showed no improvement over eyes treated with vehicle controls (FIG. 30).

In addition to targeting retinal vasculature, cells from the MLBM cell population differentiate into macrophage-like (F4/80⁺) cells, penetrate the retina, and take a position closely opposed to the retinal pigment epithelium (RPE). This localization facilitates the observed vascular and photoreceptor rescue effects of the cells from the MLBM cell population. Furthermore, once in place near the RPE, the cells from the MLBM cell population produce vascular endothelial growth factor (VEGF), as demonstrated by injection of cells from a MLBM cell population derived from a VEGF-GFP mouse, in which green fluorescent protein (GFP) is expressed upon VEGF gene activation (FIG. 31). Thus, the cells from the MLBM cell population appear to be in a VEGF "activated" state. The introduced cells from the MLBM cell population appear to recruit endogenous cells of the same type, since both GFP⁺ (introduced) and GFP- (endogenous) cells were observed in the RPE region. This localization has been observed in wild type mice during normal retinal vascular development, in rescued retinas in the *rd1*rd1* mouse and in the oxygen-induced retinopathy model.

Similar vascular targeting results were found for the MLBM cell population of Example 12. FIG. 32 shows that by P20, CD44^{hi} CD11b+ cells of Example 12 (green) specifically targeted the vasculature (red) when injected at P2, in a manner similar to the CD44-high population of Example 11. FIG. 33 shows that the CD44^{lo} CD11b⁻ of Example 12 did not specifically target the vasculature.

The MLBM cell population provides an effective and versatile treatment for ischemic retinopathic and the like ocular diseases. The cells are readily isolated from autologous bone marrow, thus minimizing potential immunogenicity often observed in cell-based therapies. Long term (up to six months) follow-up revealed only occasional rosettes and histological preservation of the neural retina in eyes injected with lineage negative cells. In addition, the MLBM cell population can be transfected with useful genes for delivering functional genes to the retina.

## Claims

1. Use of cells in the preparation of a pharmaceutical composition for treating a mammal at risk of developing or suffering from a retinopathic disease, in particular a retinopathy of prematurity, wherein:
(a) the cells are an isolated, lineage-negative, vasculotropic, myeloid-like bone marrow cell population in which the cells express the CD44 and CD 11b antigens;
(b) the cells have been transfected with a nucleic acid encoding an angiostatic fragment of tryptophanyl-tRNA synthetase (Trp-RS);
(c) the pharmaceutical composition is for administration of said cells to the retina of the mammal in an amount effective to promote physiological revascularization of damaged areas of the retina and to ameliorate damage to the retina caused by the disease.

2. The use of claim 1 wherein the angiostatic fragment of TrpRS is T2-TrpRS (SEQ ID NO: 3) or T2-TrpRS-GD (SEQ ID NO: 4).

3. The use of claim 1 wherein the cells comprise hematopoietic stem cells and endothelial progenitor cells.

4. The use of claim 1 wherein the cells are produced by a method comprising isolating bone marrow from a mammal and positively selecting cells from the bone marrow that immunoreact with an antibody selected from the group consisting of anti-CD44, anti-CD11b, and a combination thereof.

5. The use of claim 1 wherein the mammal is a human.

6. The use of claim 1 wherein the mammal is an infant mammal.

7. The use of claim 6 wherein the infant mammal has been exposed to hyperoxic conditions.

8. The use of claim 1 wherein the pharmaceutical composition is for administration of the cells by intraocular injection.

9. The use of claim 1 wherein the cells are autologous to the mammal being treated.

10. The use of claim 1 wherein the pharmaceutical composition is for administration of the cells prior to the onset of disease symptoms.

11. The use of claim 1 wherein the pharmaceutical composition is for administration of the cells prior to exposing the mammal to hyperoxic conditions.

## Patentansprüche

1. Verwendung von Zellen zur Herstellung einer pharmazeutischen Zusammensetzung für eine Behandlung eines Säugers, der ein Risiko für ein Entwickeln oder ein Leiden an einer retinopathischen Erkrankung, insbesondere Frühgeborenenretinopathie hat, wobei:
(a) die Zellen eine isolierte, Lineage-negative, vaskulotrope, myeloidähnliche Knochenmarkszellpopulation sind, worin die Zellen die CD44- und CD11b-Antigene exprimieren,
(b) die Zellen mit einer Nukleinsäure transfiziert wurden, die für ein angiostatisches Fragment von Tryptophanyl-tRNA-Synthetase (TrpRS) kodiert,
(c) die pharmazeutische Zusammensetzung einer Verabreichung der Zellen an die Retina des Säugers in einer Menge dient, die wirksam ist, um physiologische Revaskularisierung von beschädigten Bereichen der Retina zu beschleunigen und Schädigung der Retina, die durch die Erkrankung verursacht wird, zu verbessern.

2. Verwendung nach Anspruch 1, wobei das angiostatische Fragment von TrpRS T2-TrpRS (SEQ ID NO: 3) oder T2-TrpRS-GD (SEQ ID NO: 4) ist.

3. Verwendung nach Anspruch 1, wobei die Zellen hämatopoietische Stammzellen und endotheliale Vorläuferzellen umfassen.

4. Verwendung nach Anspruch 1, wobei die Zellen durch ein Verfahren erzeugt werden, das ein Isolieren von Knochenmark aus einem Säuger und eine Positivselektion von Zellen aus dem Knochenmark umfasst, die mit einem Antikörper immunoreagieren, der aus der Gruppe ausgewählt ist, bestehend aus Anti-CD44, Anti-CD11b und einer Kombination davon.

5. Verwendung nach Anspruch 1, wobei der Säuger ein Mensch ist.

6. Verwendung nach Anspruch 1, wobei der Säuger ein Säugerkind ist.

7. Verwendung nach Anspruch 6, wobei das Säugerkind hyperoxischen Bedingungen ausgesetzt wurde.

8. Verwendung nach Anspruch 1, wobei die pharmazeutische Zusammensetzung einer Verabreichung der Zellen durch intraokulare Injektion dient.

9. Verwendung nach Anspruch 1, wobei die Zellen zu dem behandelten Säuger autolog sind.

10. Verwendung nach Anspruch 1, wobei die pharmazeutische Zusammensetzung einer Verabreichung der Zellen vor dem Einsetzen von Erkrankungssymptomen dient.

11. Verwendung nach Anspruch 1, wobei die pharmazeutische Zusammensetzung einer Verabreichung der Zellen vor eine Exposition des Säugers gegenüber hyperoxischen Bedingungen dient.

## Revendications

1. Utilisation de cellules dans la préparation d'une composition pharmaceutique pour le traitement d'un mammifère risquant de développer ou de subir une affection rétinopathique, en particulier une rétinopathie de prématurité, dans laquelle:
(a) les cellules sont une population de cellules de moelle osseuse isolées, de lignée négative, vasculotropiques, analogues à un myéloïde, dans laquelle les cellules expriment les antigènes CD44 et CD11b;
(b) les cellules ont été transfectées avec un acide nucléique codant pour un fragment angiostatique de tryptophanyl-ARNt synthétase (Trp-RS);
(c) la composition pharmaceutique est pour l'administration desdites cellules à la rétine du mammifère en une quantité efficace pour favoriser la revascularisation physiologique des aires endommagées de la rétine et pour améliorer la lésion à la rétine provoquée par l'affection.

2. Utilisation selon la revendication 1, dans laquelle le fragment angiostatique de TrpRS est T2-TrpRS (SEQ ID NO: 3) ou T2-TrpRS-GD (SEQ ID NO: 4).

3. Utilisation selon la revendication 1, dans laquelle les cellules comprennent des cellules souches hématopoïétiques et des cellules progénitrices endothéliales.

4. Utilisation selon la revendication 1, dans laquelle les cellules sont produites par un procédé comprenant l'isolement de moelle osseuse à partir d'un mammifère et la sélection positive des cellules de la moelle osseuse qui donnent une immunoréaction avec un anticorps choisi dans le groupe consistant en anti-CD44, anti-CD11b, et une combinaison de ceux-ci.

5. Utilisation selon la revendication 1, dans laquelle le mammifère est un humain.

6. Utilisation selon la revendication 1, dans laquelle le mammifère est un mammifère enfant.

7. Utilisation selon la revendication 6, dans laquelle le mammifère enfant a été exposé à des conditions hyperoxiques.

8. Utilisation selon la revendication 1, dans laquelle la composition pharmaceutique est pour l'administration des cellules par injection intraoculaire.

9. Utilisation selon la revendication 1, dans laquelle les cellules sont autologues pour le mammifère en traitement.

10. Utilisation selon la revendication 1, dans laquelle la composition pharmaceutique est pour l'administration des cellules avant l'apparition des symptômes de l'affection.

11. Utilisation selon la revendication 1, dans laquelle la composition pharmaceutique est pour l'administration des cellules avant l'exposition du mammifère à des conditions hyperoxiques.


### SEQUENCE LISTING

<110> THE SCRIPPS RESEARCH INSTITUTE
<120> METHOD FOR THE TREATMENT OF RETINOPATHY OF PREMATURITY AND RELATED RETINOPATHIC DISEASES
<130> 18-216 T1
<140> EP <141> 2006-02-24
<150> 60/656,122
   <151> 2005-02-24
<160> 6
<170> FastSEQ for windows version 4.0
<210> 1
   <211> 4742
   <212> DNA
   <213> Artificial sequence
<220>
   <223> His6-tagged T2 TrpRS
<400> 1
   tggcgaatgg gacgcgccct gtagcggcgc attaagcgcg gcgggtgtgg tggttacgcg 60
   cagcgtgacc gctacacttg ccagcgccct agcgcccgct cctttcgctt tcttcccttc 120
   ctttctcgcc acgttcgccg gctttccccg tcaagctcta aatcgggggc tccctttagg 180
   gttccgattt agtgctttac ggcacctcga ccccaaaaaa cttgattagg gtgatggttc 240
   acgtagtggg ccatcgccct gatagacggt ttttcgccct ttgacgttgg agtccacgtt 300
   ctttaatagt ggactcttgt tccaaactgg aacaacactc aaccctatct cggtctattc 360
   ttttgattta taagggattt tgccgatttc ggcctattgg ttaaaaaatg agctgattta 420
   acaaaaattt aacgcgaatt ttaacaaaat attaacgttt acaatttcag gtggcacttt 480
   tcggggaaat gtgcgcggaa cccctatttg tttatttttc taaatacatt caaatatgta 540
   tccgctcatg agacaataac cctgataaat gcttcaataa tattgaaaaa ggaagagtat 600
   gagtattcaa catttccgtg tcgcccttat tccctttttt gcggcatttt gccttcctgt 660
   ttttgctcac ccagaaacgc tggtgaaagt aaaagatgct gaagatcagt tgggtgcacg 720
   agtgggttac atcgaactgg atctcaacag cggtaagatc cttgagagtt ttcgccccga 780
   agaacgtttt ccaatgatga gcacttttaa agttctgcta tgtggcgcgg tattatcccg 840
   tattgacgcc gggcaagagc aactcggtcg ccgcatacac tattctcaga atgacttggt 900
   tgagtactca ccagtcacag aaaagcatct tacggatggc atgacagtaa gagaattatg 960
   cagtgctgcc ataaccatga gtgataacac tgcggccaac ttacttctga caacgatcgg 1020
   aggaccgaag gagctaaccg cttttttgca caacatgggg gatcatgtaa ctcgccttga 1080
   tcgttgggaa ccggagctga atgaagccat accaaacgac gagcgtgaca ccacgatgcc 1140
   tgcagcaatg gcaacaacgt tgcgcaaact attaactggc gaactactta ctctagcttc 1200
   ccggcaacaa ttaatagact ggatggaggc ggataaagtt gcaggaccac ttctgcgctc 1260
   ggcccttccg gctggctggt ttattgctga taaatctgga gccggtgagc gtgggtctcg 1320
   cggtatcatt gcagcactgg ggccagatgg taagccctcc cgtatcgtag ttatctacac 1380
   gacggggagt caggcaacta tggatgaacg aaatagacag atcgctgaga taggtgcctc 1440
   actgattaag cattggtaac tgtcagacca agtttactca tatatacttt agattgattt 1500
   aaaacttcat ttttaattta aaaggatcta ggtgaagatc ctttttgata atctcatgac 1560
   caaaatccct taacgtgagt tttcgttcca ctgagcgtca gaccccgtag aaaagatcaa 1620
   aggatcttct tgagatcctt tttttctgcg cgtaatctgc tgcttgcaaa caaaaaaacc 1680
   accgctacca gcggtggttt gtttgccgga tcaagagcta ccaactcttt ttccgaaggt 1740
   aactggcttc agcagagcgc agataccaaa tactgtcctt ctagtgtagc cgtagttagg 1800
   ccaccacttc aagaactctg tagcaccgcc tacatacctc gctctgctaa tcctgttacc 1860
   agtggctgct gccagtggcg ataagtcgtg tcttaccggg ttggactcaa gacgatagtt 1920
   accggataag gcgcagcggt cgggctgaac ggggggttcg tgcacacagc ccagcttgga 1980
   gcgaacgacc tacaccgaac tgagatacct acagcgtgag ctatgagaaa gcgccacgct 2040
   tcccgaaggg agaaaggcgg acaggtatcc ggtaagcggc agggtcggaa caggagagcg 2100
   cacgagggag cttccagggg gaaacgcctg gtatctttat agtcctgtcg ggtttcgcca 2160
   cctctgactt gagcgtcgat ttttgtgatg ctcgtcaggg gggcggagcc tatggaaaaa 2220
   cgccagcaac gcggcctttt tacggttcct ggccttttgc tggccttttg ctcacatgtt 2280
   ctttcctgcg ttatcccctg attctgtgga taaccgtatt accgcctttg agtgagctga 2340
   taccgctcgc cgcagccgaa cgaccgagcg cagcgagtca gtgagcgagg aagcggaaga 2400
   gcgcctgatg cggtattttc tccttacgca tctgtgcggt atttcacacc gcatatatgg 2460
   tgcactctca gtacaatctg ctctgatgcc gcatagttaa gccagtatac actccgctat 2520
   cgctacgtga ctgggtcatg gctgcgcccc gacacccgcc aacacccgct gacgcgccct 2580
   gacgggcttg tctgctcccg gcatccgctt acagacaagc tgtgaccgtc tccgggagct 2640
   gcatgtgtca gaggttttca ccgtcatcac cgaaacgcgc gaggcagctg cggtaaagct 2700
   catcagcgtg gtcgtgaagc gattcacaga tgtctgcctg ttcatccgcg tccagctcgt 2760
   tgagtttctc cagaagcgtt aatgtctggc ttctgataaa gcgggccatg ttaagggcgg 2820
   ttttttcctg tttggtcact gatgcctccg tgtaaggggg atttctgttc atgggggtaa 2880
   tgataccgat gaaacgagag aggatgctca cgatacgggt tactgatgat gaacatgccc 2940
   ggttactgga acgttgtgag ggtaaacaac tggcggtatg gatgcggcgg gaccagagaa 3000
   aaatcactca gggtcaatgc cagcgcttcg ttaatacaga tgtaggtgtt ccacagggta 3060
   gccagcagca tcctgcgatg cagatccgga acataatggt gcagggcgct gacttccgcg 3120
   tttccagact ttacgaaaca cggaaaccga agaccattca tgttgttgct caggtcgcag 3180
   acgttttgca gcagcagtcg cttcacgttc gctcgcgtat cggtgattca ttctgctaac 3240
   cagtaaggca accccgccag cctagccggg tcctcaacga caggagcacg atcatgcgca 3300
   cccgtggcca ggacccaacg ctgcccgaga tctcgatccc gcgaaattaa tacgactcac 3360
   tatagggaga ccacaacggt ttccctctag aaataatttt gtttaacttt aagaaggaga 3420
   tatacatatg agtgcaaaag gcatagacta cgataagctc attgttcggt ttggaagtag 3480
   taaaattgac aaagagctaa taaaccgaat agagagagcc accggccaaa gaccacacca 3540
   cttcctgcgc agaggcatct tcttctcaca cagagatatg aatcaggttc ttgatgccta 3600
   tgaaaataag aagccatttt atctgtacac gggccggggc ccctcttctg aagcaatgca 3660
   tgtaggtcac ctcattccat ttattttcac aaagtggctc caggatgtat ttaacgtgcc 3720
   cttggtcatc cagatgacgg atgacgagaa gtatctgtgg aaggacctga ccctggacca 3780
   ggcctatggc gatgctgttg agaatgccaa ggacatcatc gcctgtggct ttgacatcaa 3840
   caagactttc atattctctg acctggacta catggggatg agctcaggtt tctacaaaaa 3900
   tgtggtgaag attcaaaagc atgttacctt caaccaagtg aaaggcattt tcggcttcac 3960
   tgacagcgac tgcattggga agatcagttt tcctgccatc caggctgctc cctccttcag 4020
   caactcattc ccacagatct tccgagacag gacggatatc cagtgcctta tcccatgtgc 4080
   cattgaccag gatccttact ttagaatgac aagggacgtc gcccccagga tcggctatcc 4140
   taaaccagcc ctgttgcact ccaccttctt cccagccctg cagggcgccc agaccaaaat 4200
   gagtgccagc gacccaaact cctccatctt cctcaccgac acggccaagc agatcaaaac 4260
   caaggtcaat aagcatgcgt tttctggagg gagagacacc atcgaggagc acaggcagtt 4320
   tgggggcaac tgtgatgtgg acgtgtcttt catgtacctg accttcttcc tcgaggacga 4380
   cgacaagctc gagcagatca ggaaggatta caccagcgga gccatgctca ccggtgagct 4440
   caagaaggca ctcatagagg ttctgcagcc cttgatcgca gagcaccagg cccggcgcaa 4500
   ggaggtcacg gatgagatag tgaaagagtt catgactccc cggaagctgt ccttcgactt 4560
   tcagaagctt gcggccgcac tcgagcacca ccaccaccac cactgagatc cggctgctaa 4620
   caaagcccga aaggaagctg agttggctgc tgccaccgct gagcaataac tagcataacc 4680
   ccttggggcc tctaaacggg tcttgagggg ttttttgctg aaaggaggaa ctatatccgg 4740
   at                                                                4742
<210> 2
   <211> 392
   <212> PRT
   <213> Artificial sequence
<220>
   <223> His6-tagged T2 TrpRS
<400> 2
   Met Ser Ala Lys Gly Ile Asp Tyr Asp Lys Leu Ile Val Arg Phe Gly
   1               5                   10                  15
   Ser Ser Lys Ile Asp Lys Glu Leu Ile Asn Arg Ile Glu Arg Ala Thr
               20                  25                  30
   Gly Gln Arg Pro His His Phe Leu Arg Arg Gly Ile Phe Phe Ser His
           35                  40                  45
   Arg Asp Met Asn Gln Val Leu Asp Ala Tyr Glu Asn Lys Lys Pro Phe
       50                  55                  60
   Tyr Leu Tyr Thr Gly Arg Gly Pro Ser Ser Glu Ala Met His Val Gly
   65                  70                  75                  80
   His Leu Ile Pro Phe Ile Phe Thr Lys Trp Leu Gln Asp Val Phe Asn
                   85                  90                  95
   Val Pro Leu Val Ile Gln Met Thr Asp Asp Glu Lys Tyr Leu Trp Lys
               100                 105                 110
   Asp Leu Thr Leu Asp Gln Ala Tyr Gly Asp Ala Val Glu Asn Ala Lys
           115                 120                 125
   Asp Ile Ile Ala Cys Gly Phe Asp Ile Asn Lys Thr Phe Ile Phe Ser
       130                 135                 140
   Asp Leu Asp Tyr Met Gly Met Ser Ser Gly Phe Tyr Lys Asn Val Val
   145                 150                 155                 160
   Lys Ile Gln Lys His Val Thr Phe Asn Gln Val Lys Gly Ile Phe Gly
                   165                 170                 175
   Phe Thr Asp Ser Asp Cys Ile Gly Lys Ile Ser Phe Pro Ala Ile Gln
               180                 185                 190
   Ala Ala Pro Ser Phe Ser Asn Ser Phe Pro Gln Ile Phe Arg Asp Arg
           195                 200                 205
   Thr Asp Ile Gln Cys Leu Ile Pro Cys Ala Ile Asp Gln Asp Pro Tyr
       210                 215                 220
   Phe Arg Met Thr Arg Asp Val Ala Pro Arg Ile Gly Tyr Pro Lys Pro
   225                 230                 235                 240
   Ala Leu Leu His Ser Thr Phe Phe Pro Ala Leu Gln Gly Ala Gln Thr
                   245                 250                 255
   Lys Met Ser Ala Ser Asp Pro Asn Ser Ser Ile Phe Leu Thr Asp Thr
               260                 265                 270
   Ala Lys Gln Ile Lys Thr Lys Val Asn Lys His Ala Phe Ser Gly Gly
           275                 280                 285
   Arg Asp Thr Ile Glu Glu His Arg Gln Phe Gly Gly Asn Cys Asp Val
       290                 295                 300
   Asp Val Ser Phe Met Tyr Leu Thr Phe Phe Leu Glu Asp Asp Asp Lys
   305                 310                 315                 320
   Leu Glu Gln Ile Arg Lys Asp Tyr Thr Ser Gly Ala Met Leu Thr Gly
                   325                 330                 335
   Glu Leu Lys Lys Ala Leu Ile Glu Val Leu Gln Pro Leu Ile Ala Glu
               340                 345                 350
   His Gln Ala Arg Arg Lys Glu Val Thr Asp Glu Ile Val Lys Glu Phe
           355                 360                 365
   Met Thr Pro Arg Lys Leu Ser Phe Asp Phe Gln Lys Leu Ala Ala Ala
       370                 375                 380
   Leu Glu His His His His His His
   385                 390
<210> 3
   <211> 378
   <212> PRT
   <213> Homo sapiens
<400> 3
   Ser Ala Lys Gly Ile Asp Tyr Asp Lys Leu Ile Val Arg Phe Gly Ser
   1 5 10 15
   Ser Lys Ile Asp Lys Glu Leu Ile Asn Arg Ile Glu Arg Ala Thr Gly
   20 25 30
   Gln Arg Pro His His Phe Leu Arg Arg Gly Ile Phe Phe Ser His Arg
   35 40 45
   Asp Met Asn Gln Val Leu Asp Ala Tyr Glu Asn Lys Lys Pro Phe Tyr
   50 55 60
   Leu Tyr Thr Gly Arg Gly Pro Ser Ser Glu Ala Met His Val Gly His
   65 70 75 80
   Leu Ile Pro Phe Ile Phe Thr Lys Trp Leu Gln Asp Val Phe Asn Val
   85 90 95
   Pro Leu Val Ile Gln Met Thr Asp Asp Glu Lys Tyr Leu Trp Lys Asp
   100 105 110
   Leu Thr Leu Asp Gln Ala Tyr Ser Tyr Ala Val Glu Asn Ala Lys Asp
   115 120 125
   Ile Ile Ala Cys Gly Phe Asp Ile Asn Lys Thr Phe Ile Phe Ser Asp
   130 135 140
   Leu Asp Tyr Met Gly Met Ser Ser Gly Phe Tyr Lys Asn Val Val Lys
   145 150 155 160
   Ile Gln Lys His Val Thr Phe Asn Gln Val Lys Gly Ile Phe Gly Phe
   165 170 175
   Thr Asp Ser Asp Cys Ile Gly Lys Ile Ser Phe Pro Ala Ile Gln Ala
   180 185 190
   Ala Pro Ser Phe Ser Asn Ser Phe Pro Gln Ile Phe Arg Asp Arg Thr
   195 200 205
   Asp Ile Gln Cys Leu Ile Pro Cys Ala Ile Asp Gln Asp Pro Tyr Phe
   210 215 220
   Arg Met Thr Arg Asp Val Ala Pro Arg Ile Gly Tyr Pro Lys Pro Ala
   225 230 235 240
   Leu Leu His Ser Thr Phe Phe Pro Ala Leu Gln Gly Ala Gln Thr Lys
   245 250 255
   Met Ser Ala Ser Asp Pro Asn Ser Ser Ile Phe Leu Thr Asp Thr Ala
   260 265 270
   Lys Gln Ile Lys Thr Lys Val Asn Lys His Ala Phe Ser Gly Gly Arg
   275 280 285
   Asp Thr Ile Glu Glu His Arg Gln Phe Gly Gly Asn Cys Asp Val Asp
   290 295 300
   Val Ser Phe Met Tyr Leu Thr Phe Phe Leu Glu Asp Asp Asp Lys Leu
   305 310 315 320
   Glu Gln Ile Arg Lys Asp Tyr Thr Ser Gly Ala Met Leu Thr Gly Glu
   325 330 335
   Leu Lys Lys Ala Leu Ile Glu Val Leu Gln Pro Leu Ile Ala Glu His
   340 345 350
   Gln Ala Arg Arg Lys Glu Val Thr Asp Glu Ile Val Lys Glu Phe Met
   355 360 365
   Thr Pro Arg Lys Leu Ser Phe Asp Phe Gln
   370 375
<210> 4
   <211> 378
   <212> PRT
   <213> Homo sapiens
<400> 4
   Ser Ala Lys Gly Ile Asp Tyr Asp Lys Leu Ile Val Arg Phe Gly Ser
   1               5                   10                  15
   Ser Lys Ile Asp Lys Glu Leu Ile Asn Arg Ile Glu Arg Ala Thr Gly
               20                  25                  30
   Gln Arg Pro His His Phe Leu Arg Arg Gly Ile Phe Phe Ser His Arg
           35                  40                  45
   Asp Met Asn Gln Val Leu Asp Ala Tyr Glu Asn Lys Lys Pro Phe Tyr
       50                  55                  60
   Leu Tyr Thr Gly Arg Gly Pro Ser Ser Glu Ala Met His Val Gly His
   65                  70                  75                  80
   Leu Ile Pro Phe Ile Phe Thr Lys Trp Leu Gln Asp Val Phe Asn Val
                   85                  90                  95
   Pro Leu Val Ile Gln Met Thr Asp Asp Glu Lys Tyr Leu Trp Lys Asp
               100                 105                 110
   Leu Thr Leu Asp Gln Ala Tyr Gly Asp Ala Val Glu Asn Ala Lys Asp
           115                 120                 125
   Ile Ile Ala Cys Gly Phe Asp Ile Asn Lys Thr Phe Ile Phe Ser Asp
       130                 135                 140
   Leu Asp Tyr Met Gly Met Ser Ser Gly Phe Tyr Lys Asn Val Val Lys
   145                 150                 155                 160
   Ile Gln Lys His Val Thr Phe Asn Gln Val Lys Gly Ile Phe Gly Phe
                   165                 170                 175
   Thr Asp Ser Asp Cys Ile Gly Lys Ile Ser Phe Pro Ala Ile Gln Ala
               180                 185                 190
   Ala Pro Ser Phe Ser Asn Ser Phe Pro Gln Ile Phe Arg Asp Arg Thr
           195                 200                 205
   Asp Ile Gln Cys Leu Ile Pro Cys Ala Ile Asp Gln Asp Pro Tyr Phe
       210                 215                 220
   Arg Met Thr Arg Asp Val Ala Pro Arg Ile Gly Tyr Pro Lys Pro Ala
   225                 230                 235                 240
   Leu Leu His Ser Thr Phe Phe Pro Ala Leu Gln Gly Ala Gln Thr Lys
                   245                 250                 255
   Met Ser Ala Ser Asp Pro Asn Ser Ser Ile Phe Leu Thr Asp Thr Ala
               260                 265                 270
   Lys Gln Ile Lys Thr Lys Val Asn Lys His Ala Phe Ser Gly Gly Arg
           275                 280                 285
   Asp Thr Ile Glu Glu His Arg Gln Phe Gly Gly Asn Cys Asp Val Asp
       290                 295                 300
   Val Ser Phe Met Tyr Leu Thr Phe Phe Leu Glu Asp Asp Asp Lys Leu
   305                 310                 315                 320
   Glu Gln Ile Arg Lys Asp Tyr Thr Ser Gly Ala Met Leu Thr Gly Glu
                   325                 330                 335
   Leu Lys Lys Ala Leu Ile Glu Val Leu Gln Pro Leu Ile Ala Glu His
               340                 345                 350
   Gln Ala Arg Arg Lys Glu Val Thr Asp Glu Ile Val Lys Glu Phe Met
           355                 360                 365
   Thr Pro Arg Lys Leu Ser Phe Asp Phe Gln
       370                 375
<210> 5
   <211> 423
   <212> PRT
   <213> Homo sapiens
<400> 5
   Ser Tyr Lys Ala Ala Ala Gly Glu Asp Tyr Lys Ala Asp Cys Pro Pro
   1               5                   10                  15
   Gly Asn Pro Ala Pro Thr Ser Asn His Gly Pro Asp Ala Thr Glu Ala
               20                  25                  30
   Glu Glu Asp Phe Val Asp Pro Trp Thr Val Gln Thr Ser Ser Ala Lys
           35                  40                  45
   Gly Ile Asp Tyr Asp Lys Leu Ile Val Arg Phe Gly Ser Ser Lys Ile
       50                  55                  60
   Asp Lys Glu Leu Ile Asn Arg Ile Glu Arg Ala Thr Gly Gln Arg Pro
   65                  70                  75                  80
   His His Phe Leu Arg Arg Gly Ile Phe Phe Ser His Arg Asp Met Asn
                   85                  90                  95
   Gln Val Leu Asp Ala Tyr Glu Asn Lys Lys Pro Phe Tyr Leu Tyr Thr
               100                 105                 110
   Gly Arg Gly Pro Ser Ser Glu Ala Met His Val Gly His Leu Ile Pro
           115                 120                 125
   Phe Ile Phe Thr Lys Trp Leu Gln Asp Val Phe Asn Val Pro Leu Val
       130                 135                 140
   Ile Gln Met Thr Asp Asp Glu Lys Tyr Leu Trp Lys Asp Leu Thr Leu
   145                 150                 155                 160
   Asp Gln Ala Tyr Ser Tyr Ala Val Glu Asn Ala Lys Asp Ile Ile Ala
                   165                 170                 175
   Cys Gly Phe Asp Ile Asn Lys Thr Phe Ile Phe Ser Asp Leu Asp Tyr
               180                 185                 190
   Met Gly Met Ser Ser Gly Phe Tyr Lys Asn Val Val Lys Ile Gln Lys
           195                 200                 205
   His Val Thr Phe Asn Gln Val Lys Gly Ile Phe Gly Phe Thr Asp Ser
       210                 215                 220
   Asp Cys Ile Gly Lys Ile Ser Phe Pro Ala Ile Gln Ala Ala Pro Ser
   225                 230                 235                 240
   Phe Ser Asn Ser Phe Pro Gln Ile Phe Arg Asp Arg Thr Asp Ile Gln
                   245                 250                 255
   Cys Leu Ile Pro Cys Ala Ile Asp Gln Asp Pro Tyr Phe Arg Met Thr
               260                 265                 270
   Arg Asp Val Ala Pro Arg Ile Gly Tyr Pro Lys Pro Ala Leu Leu His
           275                 280                 285
   Ser Thr Phe Phe Pro Ala Leu Gln Gly Ala Gln Thr Lys Met Ser Ala
       290                 295                 300
   Ser Asp Pro Asn Ser Ser Ile Phe Leu Thr Asp Thr Ala Lys Gln Ile
   305                 310                 315                 320
   Lys Thr Lys Val Asn Lys His Ala Phe Ser Gly Gly Arg Asp Thr Ile
                   325                 330                 335
   Glu Glu His Arg Gln Phe Gly Gly Asn Cys Asp Val Asp Val Ser Phe
               340                 345                 350
   Met Tyr Leu Thr Phe Phe Leu Glu Asp Asp Asp Lys Leu Glu Gln Ile
           355                 360                 365
   Arg Lys Asp Tyr Thr Ser Gly Ala Met Leu Thr Gly Glu Leu Lys Lys
       370                 375                 380
   Ala Leu Ile Glu Val Leu Gln Pro Leu Ile Ala Glu His Gln Ala Arg
   385                 390                 395                 400
   Arg Lys Glu Val Thr Asp Glu Ile Val Lys Glu Phe Met Thr Pro Arg
                   405                 410                 415
   Lys Leu Ser Phe Asp Phe Gln
               420
<210> 6
   <211> 401
   <212> PRT
   <213> Homo sapiens
<400> 6
   Ser Asn His Gly Pro Asp Ala Thr Glu Ala Glu Glu Asp Phe Val Asp
   1               5                   10                  15
   Pro Trp Thr Val Gln Thr Ser Ser Ala Lys Gly Ile Asp Tyr Asp Lys
               20                  25                  30
   Leu Ile Val Arg Phe Gly Ser Ser Lys Ile Asp Lys Glu Leu Ile Asn
           35                  40                  45
   Arg Ile Glu Arg Ala Thr Gly Gln Arg Pro His His Phe Leu Arg Arg
       50                  55                  60
   Gly Ile Phe Phe Ser His Arg Asp Met Asn Gln Val Leu Asp Ala Tyr
   65                  70                  75                  80
   Glu Asn Lys Lys Pro Phe Tyr Leu Tyr Thr Gly Arg Gly Pro Ser Ser
                   85                  90                  95
   Glu Ala Met His Val Gly His Leu Ile Pro Phe Ile Phe Thr Lys Trp
               100                 105                 110
   Leu Gln Asp Val Phe Asn Val Pro Leu Val Ile Gln Met Thr Asp Asp
           115                 120                 125
   Glu Lys Tyr Leu Trp Lys Asp Leu Thr Leu Asp Gln Ala Tyr Ser Tyr
       130                 135                 140
   Ala Val Glu Asn Ala Lys Asp Ile Ile Ala Cys Gly Phe Asp Ile Asn
   145                 150                 155                 160
   Lys Thr Phe Ile Phe Ser Asp Leu Asp Tyr Met Gly Met Ser Ser Gly
                   165                 170                 175
   Phe Tyr Lys Asn Val Val Lys Ile Gln Lys His Val Thr Phe Asn Gln
               180                 185                 190
   Val Lys Gly Ile Phe Gly Phe Thr Asp Ser Asp Cys Ile Gly Lys Ile
           195                 200                 205
   Ser Phe Pro Ala Ile Gln Ala Ala Pro Ser Phe Ser Asn Ser Phe Pro
       210                 215                 220
   Gln Ile Phe Arg Asp Arg Thr Asp Ile Gln Cys Leu Ile Pro Cys Ala
   225                 230                 235                 240
   Ile Asp Gln Asp Pro Tyr Phe Arg Met Thr Arg Asp Val Ala Pro Arg
                   245                 250                 255
   Ile Gly Tyr Pro Lys Pro Ala Leu Leu His Ser Thr Phe Phe Pro Ala
               260                 265                 270
   Leu Gln Gly Ala Gln Thr Lys Met Ser Ala Ser Asp Pro Asn Ser Ser
           275                 280                 285
   Ile Phe Leu Thr Asp Thr Ala Lys Gln Ile Lys Thr Lys Val Asn Lys
       290                 295                 300
   His Ala Phe Ser Gly Gly Arg Asp Thr Ile Glu Glu His Arg Gln Phe
   305                 310                 315                 320
   Gly Gly Asn Cys Asp Val Asp Val Ser Phe Met Tyr Leu Thr Phe Phe
                   325                 330                 335
   Leu Glu Asp Asp Asp Lys Leu Glu Gln Ile Arg Lys Asp Tyr Thr Ser
               340                 345                 350
   Gly Ala Met Leu Thr Gly Glu Leu Lys Lys Ala Leu Ile Glu Val Leu
           355                 360                 365
   Gln Pro Leu Ile Ala Glu His Gln Ala Arg Arg Lys Glu Val Thr Asp
       370                 375                 380
   Glu Ile Val Lys Glu Phe Met Thr Pro Arg Lys Leu Ser Phe Asp Phe
   385                 390                 395                 400
   Gln